# EUROPEAN PATENT APPLICATION

(11) **EP 4 303 297 A1**
(43) Date of publication of application: **10.01.2024**
(21) Application number: 22785040.1
(22) Date of filing: 11.04.2022
(51) Int. Cl.: C12M 3/06, C12M 3/00

(54) **MICROFLUIDIC HANGING DROP CULTURE DEVICE FOR CULTURING CELL AGGREGATES**

(30) Priority: 09.04.2021 KR 20210046483; 08.04.2022 KR 20220044172
(71) Applicant: Cellartgen Inc., Seoul 03722 (KR)
(72) Inventor: CHO, Seung Woo, Seoul 03722 (KR); KIM, Jin, Seoul 03722 (KR); KIM, Su Kyeom, Seoul 04208 (KR); CUI, Baofang, Seoul 03726 (KR)
(74) Representative: Manitz Finsterwald Patent- und Rechtsanwaltspartnerschaft mbB
(86) International application number: PCT/KR2022/005199
(87) International publication number: WO 2022/216132

(57) **Abstract**

The present invention relates to a microfluidic hanging drop culture device for culturing cell aggregates, and organoids/spheroids cultured in the device of the present invention have greater stem cell activity and higher differentiation than organoids/spheroids of a conventional culture technology. The present invention can be reused repeatedly, and can be utilized as platforms according to various uses by changing the size and number of wells, and by using a rocker in the device, a culture solution located in wells in a reservoir and a culture chamber is allowed to continuously flow through microchannels so that the environment of all of the wells is maintained to be the same, and thus cell aggregates can be cultured with high efficiency. In addition, the device for culturing cell aggregates can be used as a model for disease research and drug screening through the mass-production of cell aggregates of which disease phenotypes are maintained, and can also be used in transplantation therapy for treating diseases through the mass-production of therapeutic cell aggregates.

## Description

### TECHNICAL FIELD

The present disclosure relates to a microfluidic hanging drop culture device for culturing a cell aggregate.

### BACKGROUND

The technique for culturing a tissue-specific organoid is currently a cutting-edge field in stem cell research which is in the greatest spotlight, and the utilization thereof can be infinitely extended in the fields of regenerative medicine and new drug research such as an intractable disease model, a patient-customized drug screening platform, an in vitro model for new drug development.

Unlike the macro-scale culture, the technique for culturing cells using a microfluidic device is a technique that provides a microenvironment suitable for cells and precisely regulates culture conditions for cells which respond sensitively to the surrounding environment, and is recently gaining attention in the field of cell and tissue engineering.

However, unlike static culture, dynamic culture requires a fluid flow. Thus, for dynamic culture, there is a need for complicated equipment, such as a syringe pump or a hydraulic pump, and high proficiency of a user.

In the conventional organoid research, as a method used to impart a flow to the culture medium, there is a method of mounting a culture dish on an orbital shaker or a method of using a bioreactor such as a spinner flask. However, a nonuniform fluid flow is given to each organoid, and, thus, such methods can cause a severe batch-to-batch variation which has been pointed out as the biggest problem in organoid research.

The conventional hanging drop culture is performed by placing a microliter volume (20 µl to 50 µl) of a cell culture medium on a glass or Petri dish. Then, the glass or Petri dish is inverted. The surface tension suppresses falling of droplets, and the cells hanging in the culture medium are settled by gravity. The cells settled in the culture medium interact with adjacent cells to form a bond and form a three-dimensional spheroid or three-dimensional microstructure. The greatest difficulty in this technique is to replace the culture medium from the droplets.

In this regard, the present inventors have developed a microfluidic hanging drop culture device which is capable of generating a fluid flow without additional equipment by using a stirring device commonly used in a laboratory, and have identified that the device can enhance the survival, differentiation capability, and functionality of an organoid.

### DISCLOSURE OF THE INVENTION

### PROBLEMS TO BE SOLVED BY THE INVENTION

The present disclosure is conceived to provide a cell aggregate culture device, including: a culture chamber including one or more wells; one or more reservoirs storing a culture medium; and a microchannel connecting the culture chamber and the reservoir.

Further, the present disclosure is conceived to provide a cell aggregate culture system, including: the device; a rocker; and a culture medium shared through a microchannel.

Furthermore, the present disclosure is conceived to provide a method of culturing a cell aggregate by using the culture system.

### MEANS FOR SOLVING THE PROBLEMS

An aspect of the present disclosure provides a cell aggregate including: a culture chamber including one or more wells; one or more reservoirs for storing a culture medium; and a microchannel connecting the culture chamber and the reservoir.

In an embodiment of the present disclosure, the culture chamber may further include a microchannel connecting a plurality of wells.

In an embodiment of the present disclosure, a diameter of the well may be 1.5 mm to 4 mm, and a distance between the wells connected through the microchannel may be 1.5 mm to 5 mm.

In an embodiment of the present disclosure, the reservoir may be located at both ends of the device.

In an embodiment of the present disclosure, the cell aggregate may be a spheroid or organoid derived from one of mesenchymal stem cells, neural stem cells, vascular endothelial cells, induced pluripotent stem cells, embryonic stem cells, tissue stem cells, fetal stem cells, cancer stem cells and cardiac cells.

In an embodiment of the present disclosure, the cell aggregate may be derived from one selected from the group consisting of brain, optic cup, kidney, liver, pancreas, neural tube, stomach, large intestine, prostate, breast, heart, salivary gland, endometrium, mammary gland, thyroid, tongue, small intestine, esophagus, spinal cord, skin, bile duct, lung, blood vessel, muscle, adrenal cortex and thyroid organoids.

Another aspect of the present disclosure provides a cell aggregate culture system, including: the device; a rocker; and a culture medium shared through a microchannel.

In an embodiment of the present disclosure, the rocker may cause the device to make a swing motion.

Yet another aspect of the present disclosure provides a method of culturing a cell aggregate by using the culture system.

### EFFECTS OF THE INVENTION

A cell aggregate culture device, a cell aggregate culture system including the same, and a method of culturing a cell aggregate by using the same of the present disclosure lead to more enhanced stem cell activity and higher differentiation than the conventional cell aggregate culture technology.

The cell aggregate culture device can be reused repeatedly and can be utilized as platforms according to various uses by changing the size and number of wells.

Also, the cell aggregate culture system uses a rocker, and, thus, a culture medium located in wells inside a reservoir and a culture chamber is allowed to continuously flow through microchannels so that an environment of all of the wells is maintained to be the same, and a cell aggregate can be cultured with high uniformity and high throughput.

Further, the cell aggregate culture system can be used as a model for disease research and drug screening through mass production of cell aggregates with a well-maintained disease phenotype and can also be used in transplantation therapy for treating diseases through mass production of therapeutic cell aggregates.

### BRIEF DESCRIPTION OF THE DRAWINGS

**FIG.** 1 shows the structure of a hanging drop chip (HD chip) of the present disclosure.
**FIG. 2** and **FIG.** 3 show the structure and culture method of the HD chip.
**FIG. 4** shows photos showing the formation and culturing of spheroids by using the HD chip.
**FIG. 5** shows the reusability of the HD chip formed of PDMS.
**FIG. 6** shows photos showing various methods of forming a cell aggregate by using the HD chip.
**FIG. 7** shows photos showing the results of culturing adipose stem cell spheroids (hADSC spheroids) for comparison with the conventional Petri dish hanging drop culture method.
**FIG. 8A** shows photos showing the results of culturing adipose stem cell spheroids (hADSC spheroids) for comparison with the conventional U-bottom well-plate culture method. **FIG. 8B** shows the results of culturing adipose stem cell spheroids (hADSC spheroids) for comparison with the conventional U-bottom well-plate culture method. **FIG. 8C** shows the results of culturing adipose stem cell spheroids (hADSC spheroids) for comparison with the conventional U-bottom well-plate culture method.
**FIG. 9A** shows photos showing the results of culturing neural stem cell spheroids (hNSC spheroids) as various examples of spheroid/organoid culture application. **FIG. 9B** shows the results of culturing neural stem cell spheroids (hNSC spheroids) as various examples of spheroid/organoid culture application.
**FIG. 10** shows photos showing the results of culturing cardiac spheroids as various examples of spheroid/organoid culture application.
**FIG. 11A** shows photos showing the results of culturing brain organoids (human iPSC-derived brain organoids) as various examples of spheroid/organoid culture application. **FIG. 11B** shows photos showing the results of culturing brain organoids (human iPSC-derived brain organoids) as various examples of spheroid/organoid culture application. **FIG. 11C** shows the results of culturing brain organoids (human iPSC-derived brain organoids) as various examples of spheroid/organoid culture application.
**FIG. 12A** shows the result of analyzing the difference in differentiation ability depending on the cell composition of human iPSC-derived liver organoids cultured in the HD chip. **FIG. 12B** shows the result of analyzing the difference in differentiation ability depending on the cell composition of human iPSC-derived liver organoids cultured in the HD chip.
**FIG. 13** shows the result of comparing the long-term culture differentiation ability of human iPSC-derived liver organoids (HEM).
**FIG. 14** and **FIG. 15** show the results of analyzing the expression of markers in human iPSC-derived liver organoids (HEM).
**FIG. 16A** shows the result of analyzing the expression of markers and functionality of human iPSC-derived liver organoids. **FIG. 16B** shows the result of analyzing the expression of markers and functionality of human iPSC-derived liver organoids.
**FIG. 17** shows the result of comparing the expression of markers during long-term culture of human iPSC-derived liver organoids.
**FIG. 18** and **FIG. 19** show the result of fabricating a high-throughput HD chip.
**FIG. 20A** shows the result of fabricating a high-throughput HD chip. **FIG. 20B** shows the result of fabricating a high-throughput HD chip. **FIG. 20C** shows the result of fabricating a high-throughput HD chip.
**FIG. 21A** shows the result of analyzing the uniformity of human iPSC-derived liver organoids produced in a 100-well HD chip. **FIG. 21B** shows the result of analyzing the uniformity of human iPSC-derived liver organoids produced in a 100-well HD chip.

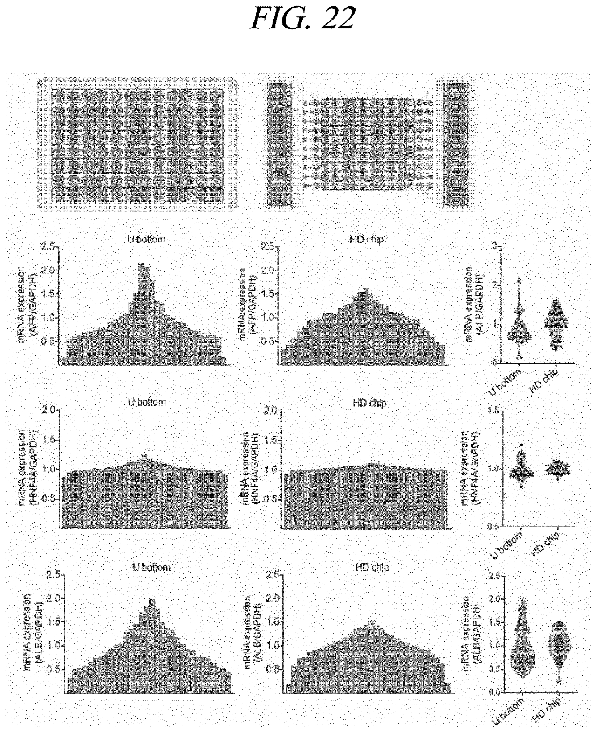

**FIG. 22** shows the result of comparing the uniformity in gene expression of human iPSC-derived liver organoids cultured in a high-throughput HD chip.
**FIG. 23A** shows the result of analyzing the mass production and functionality of human iPSC-derived normal and nonalcoholic steatohepatitis organoids. **FIG. 23B** shows the result of analyzing the mass production and functionality of human iPSC-derived normal and nonalcoholic steatohepatitis organoids.
**FIG. 24A** shows mass production of human iPSC-derived nonalcoholic steatohepatitis organoids and the result of effective drug test. **FIG. 24B** shows mass production of human iPSC-derived nonalcoholic steatohepatitis organoids and the result of effective drug test.
**FIG. 25A** shows mass production of human iPSC-derived nonalcoholic steatohepatitis organoids and the result of ROS quantitative analysis. **FIG. 25B** shows mass production of human iPSC-derived nonalcoholic steatohepatitis organoids and the result of ROS quantitative analysis.
**FIG. 26A** compares the results of mass culture of human iPSC-derived pancreatic organoids cultured by the conventional microwell and U-bottom well-plate methods. **FIG. 26B** compares the results of mass culture of human iPSC-derived pancreatic organoids cultured by the conventional microwell and U-bottom well-plate methods.
**FIG. 27A** shows the results of comparing the expression of differentiation markers ability of human iPSC-derived pancreatic organoids **FIG. 27B** shows the results of comparing the gene expression levels and differentiation ability of human iPSC-derived pancreatic organoids.
**FIG. 28** shows the result of comparing insulin production of human iPSC-derived pancreatic organoids.
**FIG. 29** shows the result of fusion of spheroids (human adipose stem cell (hADSC) spheroids) by chip-to-chip transfer.
**FIG. 30A** shows the result of comparing heterogeneous organoid fusion of human iPSC-derived liver and pancreas organoids. **FIG. 30B** shows the result of comparing heterogeneous organ fusion of human iPSC-derived liver-pancreas fused organoids. **FIG. 30C** shows the result of comparing heterogeneous organoid fusion of human iPSC-derived liver and pancreas organoids. FIG. 30D shows the result of comparing heterogeneous organoid fusion of human iPSC-derived liver and pancreas organoids.
**FIG. 31** shows the result of high-throughput fusion efficiency of hADSC spheroids by using a fabricated high-throughput HD chip.
**FIG. 32** shows the results of high-throughput fusion efficiency of liver-pancreatic organoids in a HD chip.
**FIG. 33A** shows the result of transplantation of a human iPSC-derived liver organoid cultured in a HD chip into a mouse steatohepatitis model. **FIG. 33B** shows the result of transplantation of a human iPSC-derived liver organoid cultured in a HD chip into a mouse steatohepatitis model.
**FIG. 34A** shows the result of transplantation of human iPSC-derived liver organoids cultured in a HD chip into a mouse steatohepatitis model. **FIG. 34B** shows the result of transplantation of a human iPSC-derived liver organoid cultured in a HD chip into a mouse steatohepatitis model. **FIG. 34C** shows the result of transplantation of a human iPSC-derived liver organoid cultured in a HD chip into a mouse steatohepatitis model. **FIG. 34D** shows the result of transplantation of human iPSC-derived liver organoids cultured in a HD chip into a mouse steatohepatitis model.
**FIG. 35** shows the result of on-chip drug screening, and fluorescence quantitative analysis (hADSC-human adipose stem cell spheroid) using a plate reader.
**FIG. 36** shows the result of fabricating a HD chip prototype by 3D printing.
**FIG. 37** shows the result of culturing a three-dimensional spheroid/organoid by using an accessory.
**FIG. 38** shows the result of culturing a multilayer three-dimensional adipose stem cell (ADSC) spheroid using an accessory.

### BEST MODE FOR CARRYING OUT THE INVENTION

An aspect of the present disclosure provides a cell aggregate including: a culture chamber including one or more wells; one or more reservoirs for storing a culture medium; and a microchannel connecting the culture chamber and the reservoir.

The culture chamber may include one or more wells, and the wells may be filled with a culture medium to culture a cell aggregate. The wells of the culture chamber may be arranged in a row, or may be arranged in a plurality of rows in consideration of the culture scale or use.

The reservoir is a device for supplying and sharing a culture medium to and with the culture chamber through the microchannel, and may be fabricated in various shapes and numbers in consideration of the purpose of a test.

The microchannel is configured to connect the culture chamber and the reservoir, and the shape, size, length, etc. of the microchannel can be modified without limitation as long as the culture medium can flow therein.

In an embodiment of the present disclosure, the culture chamber may further include a microchannel connecting a plurality of wells. Since the culture chamber includes one or more wells, it may further include a microchannel between the wells in addition to the microchannel between the culture chamber and the reservoir to share the culture medium between the plurality of wells and the reservoir and maintain a constant environment. Meanwhile, the microchannel between the plurality of wells may be formed only between some wells in consideration of the purpose of some tests.

In an embodiment of the present disclosure, a diameter of the well may be 1.5 mm to 4 mm, specifically 2 mm to 3.1mm, and a distance between the wells connected through the microchannel may be 1.5 mm to 5 mm, specifically 2.5 mm to 4.5 mm. More specifically, the diameter of the well may be 2.0 mm or 3.1 mm, the distance between the wells connected through the microchannel may be 2.5 mm or 4.5 mm. Most specifically, when the diameter of the well is 2.0 mm, the distance between the wells is 2.5 mm, and when the diameter of the well is 3.1 mm, the distance between the wells may be 4.5 mm.

In an embodiment of the present disclosure, the reservoir may be located at both ends of the device. The reservoir may be located at both ends of the device and connected to one or more wells of the culture chamber through the microchannel. Also, the wells may be connected through the microchannel. Therefore, the reservoir, at least one well and the reservoir may be connected to each other. Thus, the culture medium may be supplied to and shared by the all of wells.

In an embodiment of the present disclosure, the cell aggregate may be a spheroid or organoid derived from one of mesenchymal stem cells, neural stem cells, vascular endothelial cells, induced pluripotent stem cells, embryonic stem cells, tissue stem cells, fetal stem cells, cancer stem cells and cardiac cells.

The term "spheroid" refers to a spherical cell aggregate. The substantially spherical shape is not limited to a perfectly spherical one, but may include a slightly flattened shape.

The term "organoid" refers to an ultraminiature body organ obtained by culturing cells derived from the tissue or pluripotent stem cells in a 3D form to produce a form such as an artificial organ. The organoid is a three-dimensional tissue analog that contains organ-specific cells which originate from stem cells and self-organize (or self-pattern) in a similar manner to the in vivo condition. The organoid can be developed into a specific tissue by restricted element (e.g., growth factor) patterning. The organoid can have the original physiological characteristics of the cells and can have an anatomical structure that mimics the original state of a cell mixture (including all remaining stem cells and the neighboring physiological niche as well as limited cell types). A three-dimensional culture method allows the organoid to be better arranged in terms of cell to cell functions, and to have an organ-like form with functionality and a tissue-specific function.

In an embodiment of the present disclosure, the cell aggregate may be derived from one selected from the group consisting of brain, optic cup, kidney, liver, pancreas, neural tube, stomach, large intestine, prostate, breast, heart, salivary gland, endometrium, mammary gland, thyroid, tongue, small intestine, esophagus, spinal cord, skin, bile duct, lung, blood vessel, muscle, adrenal cortex and thyroid organoids.

Another aspect of the present disclosure provides a cell aggregate culture system, including the culture device; a rocker; and a culture medium that is shared through a microchannel.

The "rocker" is capable of imparting a dynamic flow to the culture medium by causing the culture device to make a motion at a constant cycle. The rocker is capable of imparting a dynamic flow to the culture medium by changing the position of the device, and the range or form of the motion is not particularly limited.

The term "culture medium" is a culture medium for cells and is, at the same time, a medium for transporting nutrients, oxygen, or the like. The culture medium is capable of supplying nutrients, oxygen, or the like necessary for cells and removing waste.

In an embodiment of the present disclosure, the rocker may cause the device to make a swing motion.

The term "swing motion" refers to an operation mode of a mechanical device, indicating a motion in which the driving part does not rotate around an axis but reciprocates over a certain section.

The device makes a swing motion at a constant cycle. Thus, the culture medium in the device can make a reciprocating motion in the chamber at a constant cycle, and an environment in which the cell aggregate can be stably cultured can be established.

Yet another aspect of the present disclosure provides a method of culturing a cell aggregate by using the culture system.

The culture refers to a process of maintaining and growing cells under suitable conditions, and the suitable conditions may refer to, for example, the temperature at which the cells are maintained, nutrient availability, atmospheric CO₂ content, and cell density.

Appropriate culture conditions for maintaining, proliferating, expanding, and differentiating different types of cells are known in the art and are documented. Suitable conditions for formation of the cell aggregate may be conditions that facilitate or allow for cell differentiation and formation of a multicellular structure.

### MODE FOR CARRYING OUT THE INVENTION

Hereinafter, one or more embodiments of the present disclosure will be described in further detail with reference to the following examples. These examples are for illustrative purposes only and are not intended to limit the scope of the one or more embodiments of the present disclosure.

### Example 1: Fabrication of organoid culture device

A chip composed of a culture chamber in which organoids are to be cultured and a reservoir to contain a culture medium was implemented, and a diameter of a well and a distance between wells can be selected according to the purpose of a test and the characteristics of an organoid. Specifically, if the purpose of the test is to form a high-throughput organoid, the culture efficiency can be increased by reducing the diameter of the well and the distance between the wells. If the purpose of the test is to analyze an organoid as well as to form the organoid, a commercial plate reader standardized for an existing plate may be used

Meanwhile, it is possible to maintain a flow of a culture medium and culture medium droplets to confine cells by using the surface tension of the culture medium, and a HD chip was basically fabricated with a PDMS polymer by a general method to fabricate a microfluidic chip.

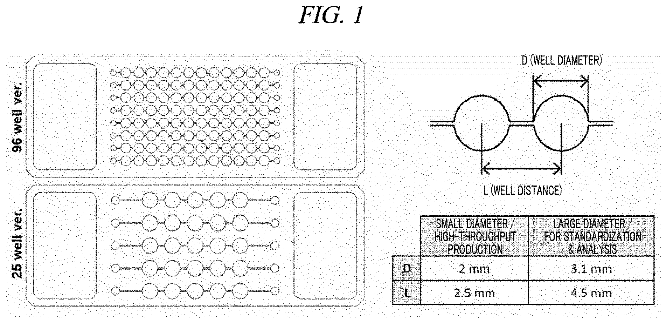

Specifically, the HD chip is fabricated by making a desired plate design (FIG. 1) and fabricating a silicon wafer with an embossed pattern by a lithography process, and a device pattern PDMS is prepared by a soft lithography process for curing PDMS (polydimethylsiloxane) by using the silicon wafer as a template. After shaping with a blade, a biopsy punch is used to punch a hole in a well chamber or a culture medium inlet. The devices prepared as described above are activated by irradiating the surfaces with oxygen plasma at 60 W for 1 minute, and then attached to each other. For complete bonding, it is placed in an oven over 70°C overnight and then sterilized through high-temperature autoclaving and UV irradiation.

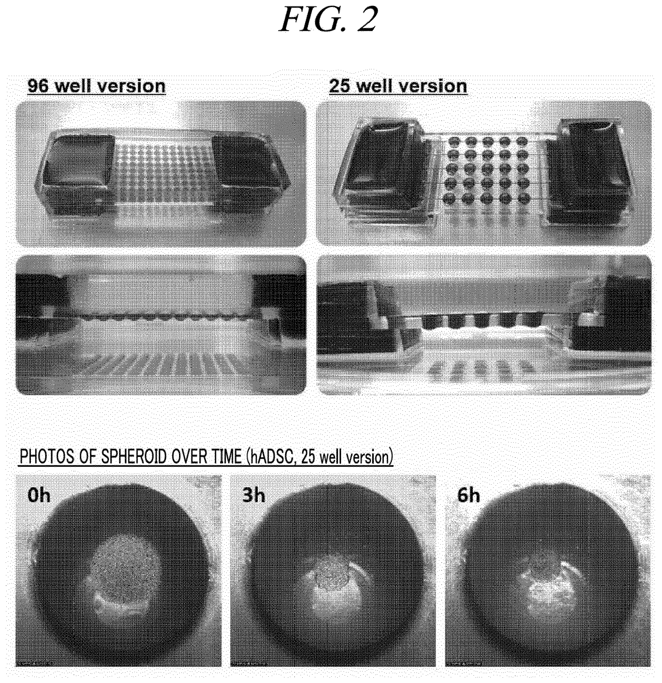

As a result, a 96-well device and a 25-well device were fabricated as shown at the top of **FIG. 2****.**

Also, it was confirmed that when human adipose stem cells (hADSCs) were cultured in the fabricated device, the cells were aggregated to form a spheroid within one day (within 6 hours in the case of hADSCs).

Specifically, the hADSCs were placed and cultured in the 25-well device prepared in Example 1.

When a cell suspension was injected into the 25-well device, the cells sank by gravity and gathered at the end of the culture medium droplets. Then, the cells were aggregated over time to form a spheroid/organoid at a density of 6000 cells/organoid (bottom of **FIG.** 2).

Meanwhile, the device of the present disclosure may use a rocker to continuously mix a culture medium in both culture medium reservoirs with a culture medium in each culture medium and thus replace the consumed culture medium and the secreted waste.

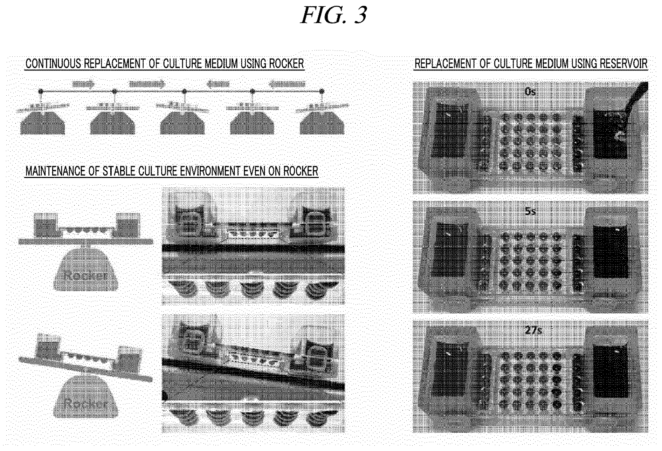

Accordingly, it was confirmed that the culture medium droplet structure is stably maintained in the HD chip even on the rocker, and the culture medium can be easily replaced through the reservoirs on both sides **(****FIG.** 3).

### Test Example 1: Formation of spheroid using HD chip and confirmation of culture result

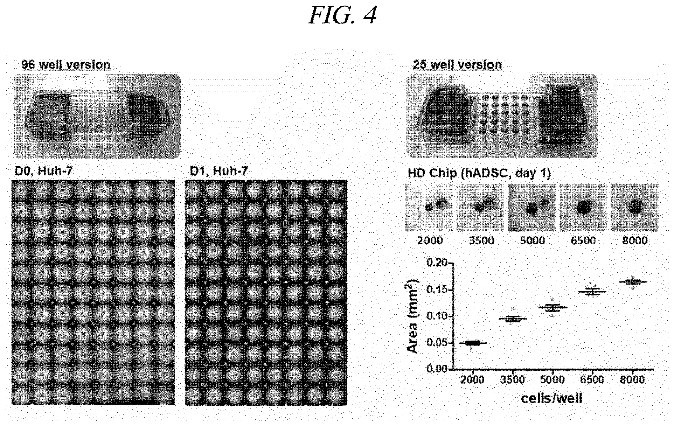

It was confirmed that it can be used as a high-throughput spheroid/organoid formation platform by increasing the number of culture chambers (96 wells), (left of **FIG.** 4). Accordingly, it is possible to easily inject cells at once to mass produce spheroids/organoids.

As can be seen from the right side of **FIG.** 4, spheroids uniform in size can be formed by using the HD chip, and the size of the spheroids can be adjusted according to the number of injected cells. Also, the spheroids can be stably cultured without being attached to the surface of the device or being deformed during long-term culture from the initial stage of spheroid formation.

### Test Example 2: Confirmation of reusability of HD chip fabricated with PDMS

The reusability of the HD chip fabricated with PDMS was confirmed.

A HD chip can be fabricated with various materials. Particularly, if the HD chip is fabricated with PDMS, it can be sterilized and then reused.

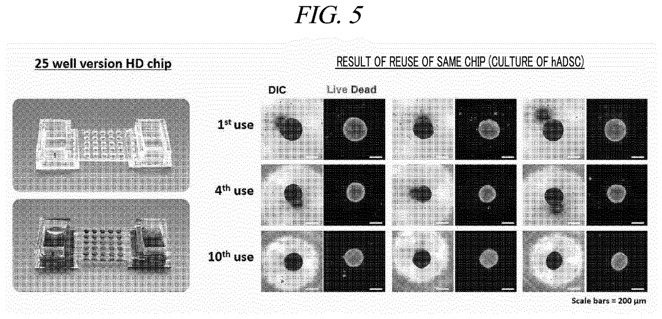

The HD chip was fabricated with PDMS and then reused more than 10 times for culturing hADSC spheroids. Even in this case, the hADSC spheroids were well formed each time, showing high cell viability without apoptosis (FIG. 5).

### Test Example 3: Confirmation of various cell aggregate formation methods using HD chip (Formation of 3D spheroid and organoid by various on-chip cell/gel loading methods)

Most of the conventional spheroid/organoid culture systems are designed to perform simple three-dimensional culture, but the HD chip system developed in the present disclosure can perform various types of on-chip three-dimensional culture that are difficult to implement with the conventional culture systems.

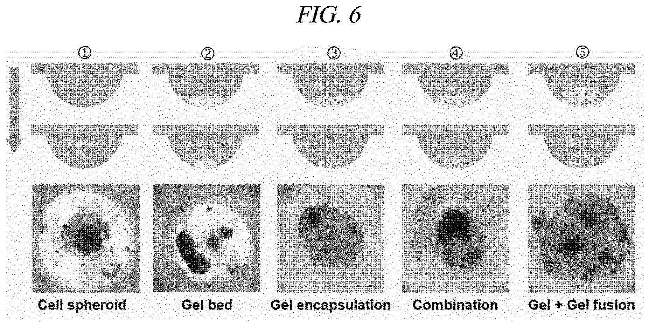

Specifically, it was confirmed that various types of three-dimensional cell aggregate culture, such as (1) cell spheroid (cell spheroid), (2) culture on hydrogel (gel bed), (3) culture in hydrogel (gel encapsulation), (4) mixture of culture methods (combination), (5) heterogeneous hydrogel fusion (gel+gel fusion), etc., can be performed (FIG. 6).

Also, cells can be further injected during culture. Thus, it is expected that advanced spheroids and organoids composed of various types of cells will be fabricated.

### Test Example 4: Comparison with conventional culture method

### Test Example 4-1. Comparison with conventional Petri dish hanging drop culture method (culture of human adipose stem cell spheroid (hADSC spheroid))

A conventional hanging drop culture method using a Petri dish lid, which has been generally used, requires skill to form uniform culture medium droplets. Also, it is very cumbersome to replace a culture medium. Further, a total amount of the culture medium is small, and, thus, cells sensitive to a culture environment and cells requiring time to form a spheroid/organoid show low viability.

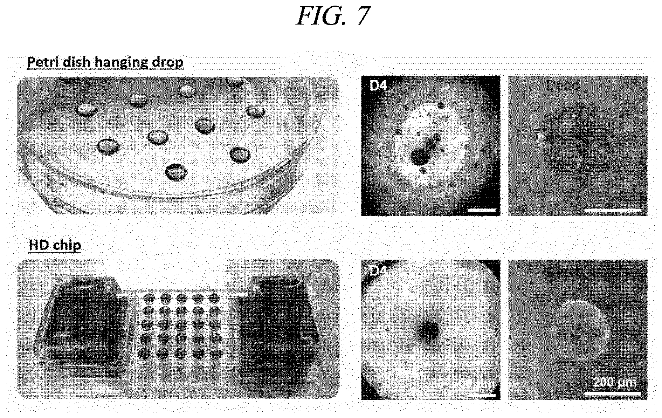

However, with the HD chip stably supplied with a culture medium through a microchannel, a uniform spheroid/organoid with improved cell viability and activity can be formed as shown in **FIG. 7****.**

### Test Example 4-2. Comparison with conventional U-bottom well-plate culture method (culture of human adipose stem cell spheroid (hADSC spheroid))

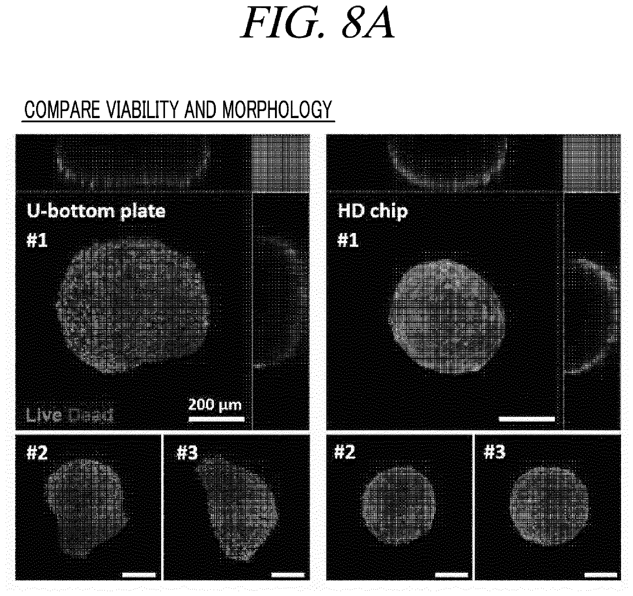

A currently widely used U-bottom well-plate showed high cell viability unlike the above-described hanging drop culture dish, but as a result of live/dead assay after 6 days of culture, a spheroid had an irregular morphology and a relatively sparse structure, which is thought to be due to contact with the floor surface. However, it was confirmed that a human adipose stem cell spheroid cultured in the HD chip without a floor surface, which cells can contact with or can be attached to, had a uniform spherical shape, high cell density, and excellent intercellular junction (FIG. 8A).

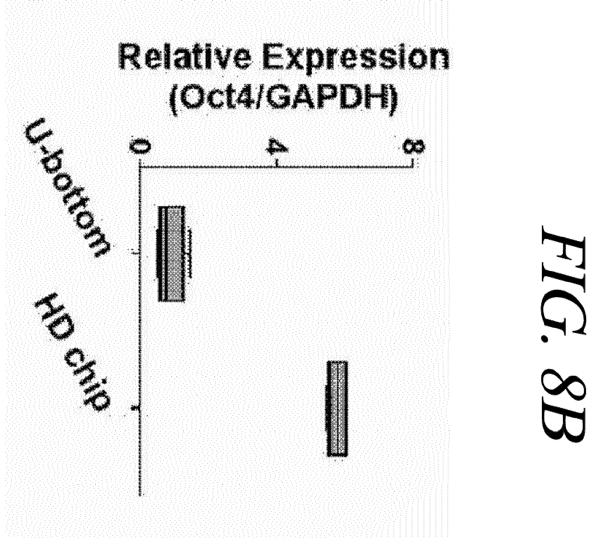

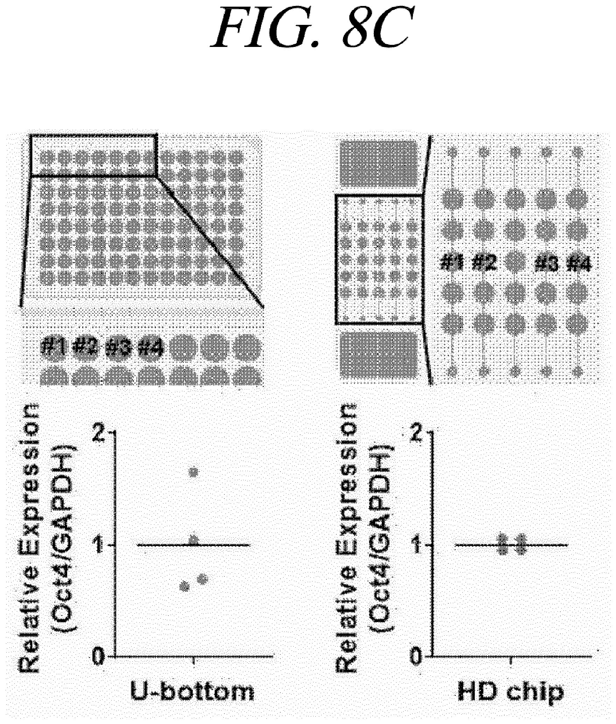

As a result of qPCR analysis for a stemness marker (Oct4) by extracting mRNA from the spheroids cultured in the respective wells of the U-bottom plate and the spheroids cultured in the respective wells of the HD chip, it was confirmed that the expression level of Oct4 was significantly improved (FIG. 8B) and the uniformity of expression was greatly improved in the HD chip group compared to the U-bottom group (FIG. 8C).

Therefore, it can be seen from these results that the HD chip culture system can fabricate a stem cell spheroid of much better quality than the conventional systems.

### Test Example 5: Culture of various spheroids/organoids

### Test Example 5-1. Culture of neural stem cell spheroid (hNSC spheroid)

Human neural stem cells (hNSCs) were naturally aggregated to each other to form a spheroid through suspension culture in a Petri dish. Spheroids are randomly formed in the Petri dish and thus have various sizes and shapes, which results in low uniformity. Thus, it is known that there is a very large difference between batches in proliferation and differentiation ability of the cells constituting each spheroid.

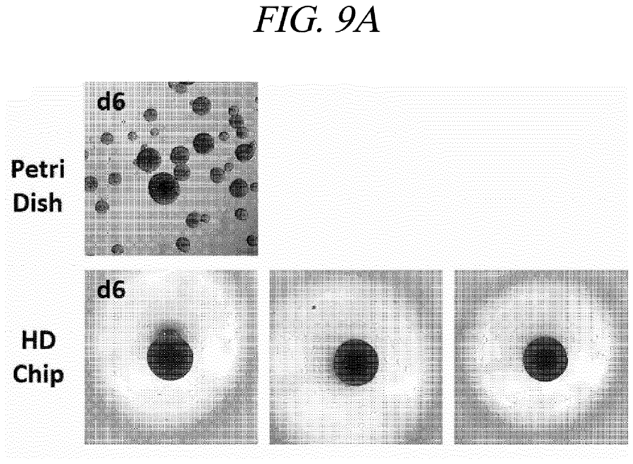

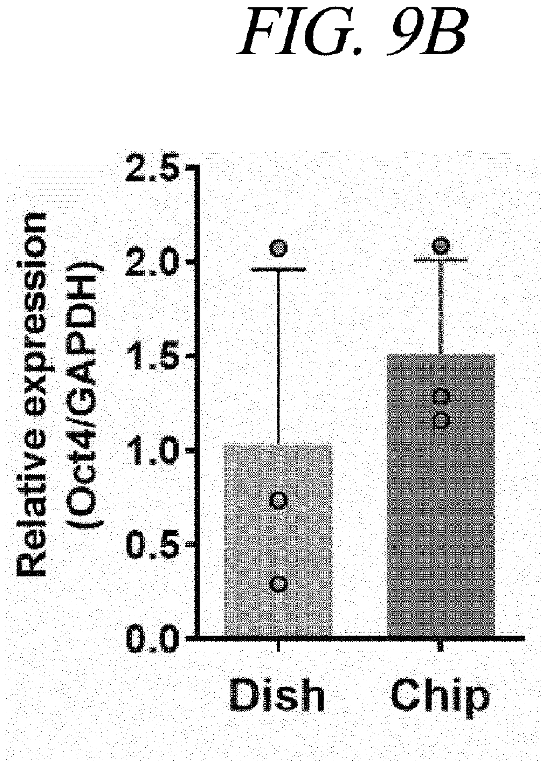

As a result of comparing and analyzing neural stem cell spheroids cultured in the same Petri dish and neural stem cell spheroids cultured in the same HD chip, it was confirmed that the spheroids cultured in the HD chip were formed to have a more uniform size (FIG. 9A). When qPCR was performed on day 6 of culture to analyze the expression of a stemness marker (Oct4), it was confirmed that the expression of Oct4 in each spheroid formed in the HD chip was more uniform than in the spheroids formed in the Petri dish (FIG. 9B).

### Test Example 5-2. Culture of cardiac spheroid

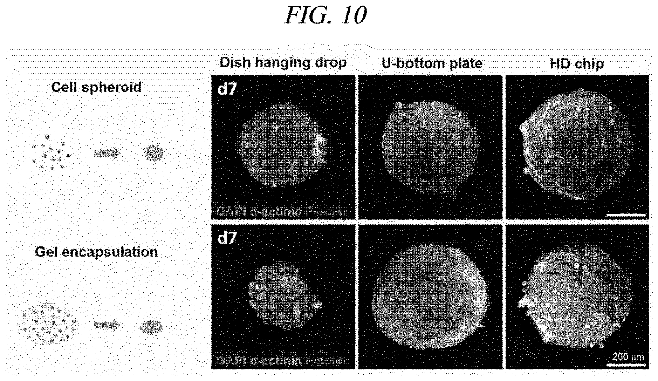

**A** HD chip culture system was applied to fabricate a cardiac spheroid using cardiomyocytes induced by direct reprogramming from mouse fibroblasts. As a result, as can be seen from FIG. 10, the cardiac spheroid formed through culture in the HD chip was structurally more developed than spheroids formed through hanging drop culture in a conventional Petri dish or culture in a conventional U-bottom well-plate. Also, the expression of a cardiomyocyte marker (a-actinin) in the cardiac spheroid formed through culture in the HD chip was greatly improved with a clear α-actinin pattern.

Further, an α-actinin structure was more clearly formed and a more patterned arrangement with adjacent cells was present in the cardiac spheroid cultured in the HD chip, when using the HD chip. Accordingly, it can be seen that the HD chip can fabricate a cardiac spheroid having electrophysiologically higher maturity and functionality, such as heartbeat, than the conventional methods.

### Test Example 5-3. Culture of human iPSC-derived brain organoid

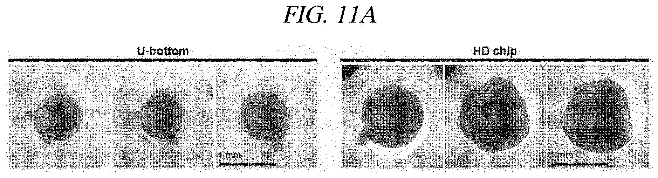

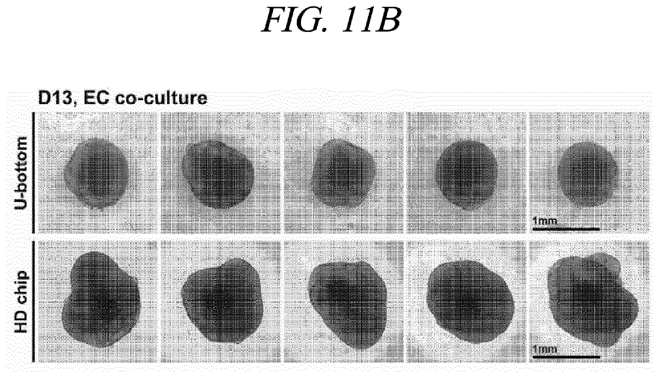

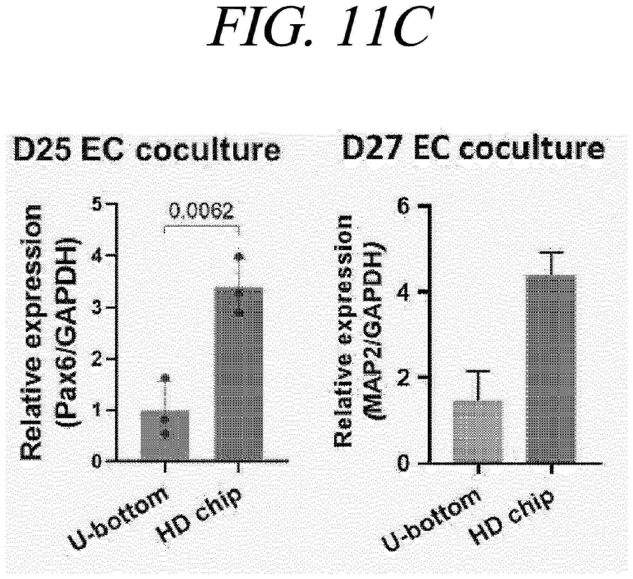

While human iPSC-derived brain organoids were formed and cultured under respective conditions, the shapes of the organoids (FIG. **11A** and **FIG.** 11B) and the result of qPCR for analyzing the expression of neuronal differentiation markers (FIG. 11C) were confirmed (Pax6 - day 25 of culture and MAP2 - day 27 of culture).

It was confirmed that a size of the brain organoid cultured in the HD chip was greater than that of the brain organoid cultured in the U-bottom well-plate, which is a control group (FIG. **11A** and **FIG.** 11B). Also, as a result of checking the gene expression in the brain organoid co-cultured with endothelial cells (EC) in the HD chip, it was confirmed that the expression of a neuronal precursor cell marker (Pax6) and a neuronal cell marker (MAP2) was significantly increased (FIG. 11C).

### Test Example 6: Confirmation of culture of human iPSC-derived liver organoid

### Test Example 6-1. Analysis of difference in differentiation ability depending on cell composition of liver organoid cultured in HD chip

**To** analyze the difference in differentiation ability between two types of human iPSC-derived liver organoids cultured in the HD chip (HM - iPSC-derived hepatocytes (H): mesenchymal stem cells (M) = 10:2, HEM-iPSC-derived hepatocytes (H): vascular endothelial cells (E):mesenchymal stem cells (M)=10:7:2), the marker expression was compared and analyzed by immunostaining and quantitative PCR (qPCR) on day 5 and day 7 of culture.

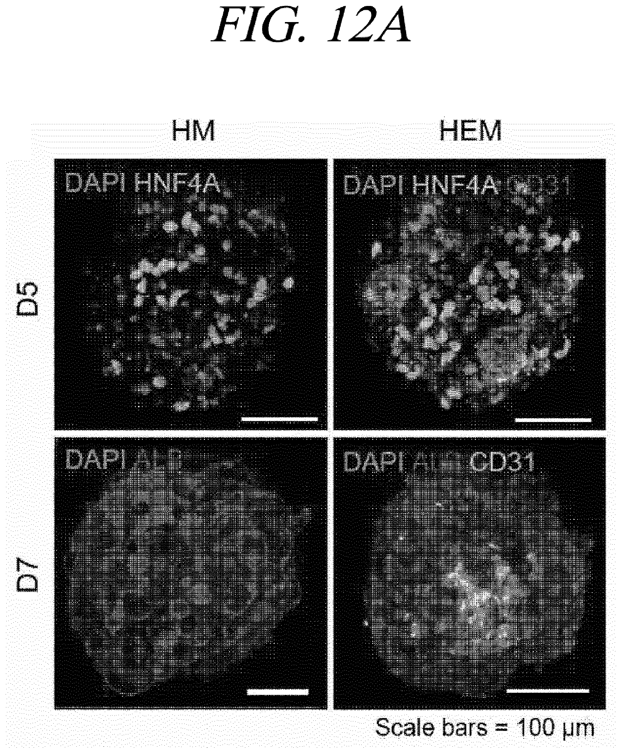

As a result, as shown in **FIG. 12A****,** it was confirmed that HNF4A and ALB, liver-specific differentiation markers were well expressed in both the HM and HEM liver organoids cultured in the HD chip, and CD31, a endothelial cell marker, was also well expressed in the HEM.

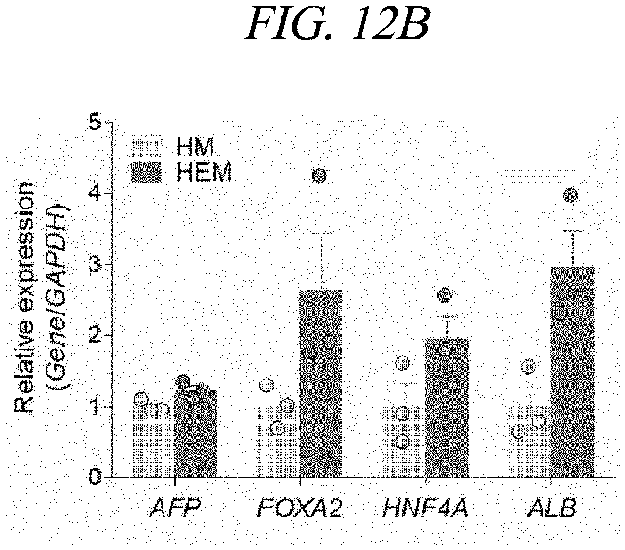

Also, as shown in **FIG. 12B****,** when the HM and HEM groups were compared in terms of gene expression on day 7 of culture, the expression of hepatic differentiation markers (AFP, FOXA2, HNF4A, ALB) tended to increase in the HEM group containing vascular endothelial cells. Thus, it was confirmed that the liver organoid containing vascular endothelial cells developed into a more mature liver organoid.

It can be seen from these results that the HD chip can efficiently culture a liver organoid composed of various cells.

### Test Example 6-2. Comparison of long-term culture differentiation of human iPSC-derived liver organoid (HEM)

Human iPSC-derived liver organoids (HEM) containing vascular endothelial cells were cultured for 20 days in respective culture systems and then, the gene expression for various markers was compared.

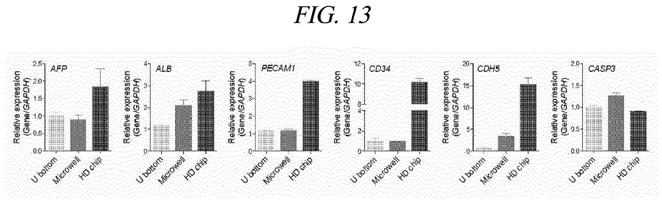

As a result, as shown in **FIG.** 13, the differentiation markers showed a tendency to significantly increase in the liver organoid cultured in the HD chip compared to the control groups, the U-bottom plate and the microwell. The expression of hepatic differentiation-related markers (AFP, ALB) and vascular markers (PECAM1, CD34, CDH5) increased in the HD chip group, and CASP3, an apoptosis marker, showed a tendency to slightly decrease in the HD chip group.

Therefore, the HD chip can improve hepatic differentiation and vascular maturation of the liver organoid (HEM) and reduce apoptosis compared to the conventional organoid culture systems, the U-bottom well-plate and the microwell. Thus, the HD chip can fabricate a liver organoid of better quality.

### Test Example 6-3. Analysis of marker expression in human iPSC-derived liver organoid (HEM)

Human iPSC-derived liver organoids (HEM) containing vascular endothelial cells were cultured for 15 days in respective culture systems and then, the expression of hepatic differentiation markers and endothelial cell markers was compared between groups by immunostaining.

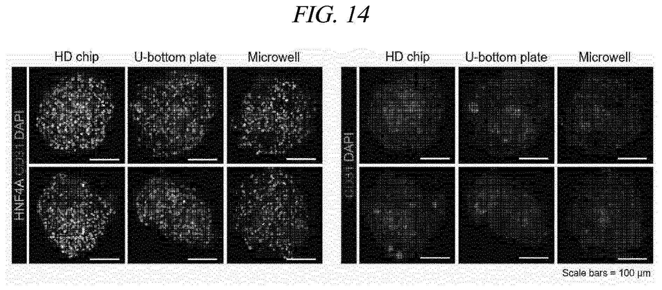

As a result, as shown in **FIG.** 14, the expression level of HNF4A, a hepatic differentiation marker, was higher in the liver organoid cultured in the HD chip than in the control groups, the U-bottom plate and the microwell, and the expression of CD31, a endothelial cell marker, was further improved in the liver organoid cultured in the HD chip. It was confirmed that the shape and morphology of the liver organoid cultured in the HD chip were more uniform than in the control groups.

Therefore, it was confirmed that the HD chip can improve hepatic differentiation and vascular maturation of the liver organoid (HEM) compared to the conventional organoid culture systems, the U-bottom well-plate and the microwell. Thus, it was confirmed that the HD chip can fabricate a liver organoid of excellent quality.

Human iPSC-derived liver organoids (HEM) containing vascular endothelial cells were cultured for 15 days in respective culture systems and then, the expression of hepatic differentiation markers and endothelial cell markers was compared between groups by immunostaining.

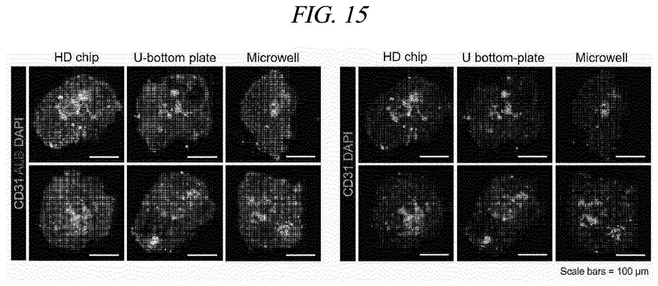

As a result, as shown in **FIG. 15****,** the expression level of ALB, a hepatic differentiation marker, was higher in the liver organoid cultured in the HD chip than in the control groups, the U-bottom plate and the microwell, and the expression of CD31, a endothelial cell marker, was further improved in the liver organoid cultured in the HD chip. It was confirmed that the shape and morphology of the liver organoid cultured in the HD chip were more uniform than in the control groups.

Therefore, it was confirmed that the HD chip can improve hepatic differentiation and vascular maturation of the liver organoid (HEM) compared to the conventional organoid culture systems, the U-bottom well-plate and the microwell. Thus, it was confirmed that the HD chip can fabricate a liver organoid of excellent quality.

Test Example 6-4. Analysis of marker expression and functionality of human iPSC-derived liver organoid

Human iPSC-derived liver organoids were cultured for 15 days in respective culture systems and then, the marker expression was compared between groups by immunostaining and a urea synthesis ability, which is one of the important indicators of hepatic function was compared between the groups.

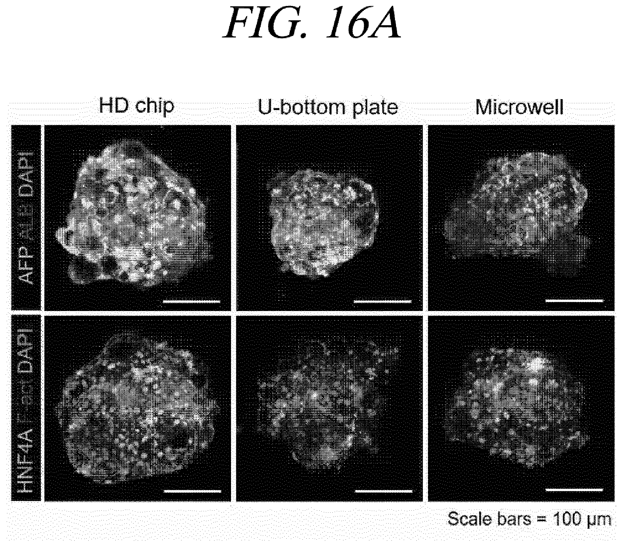

As a result, as shown in **FIG. 16A****,** the expression levels of hepatic differentiation markers, AFP and HNF4A, were higher in the liver organoid cultured in the HD chip than in the control group U-bottom well-plate and microwell. Also, it can be seen that when an actin fiber structure was checked by F-actin staining, an organoid was formed more uniformly in shape in the HD chip group.

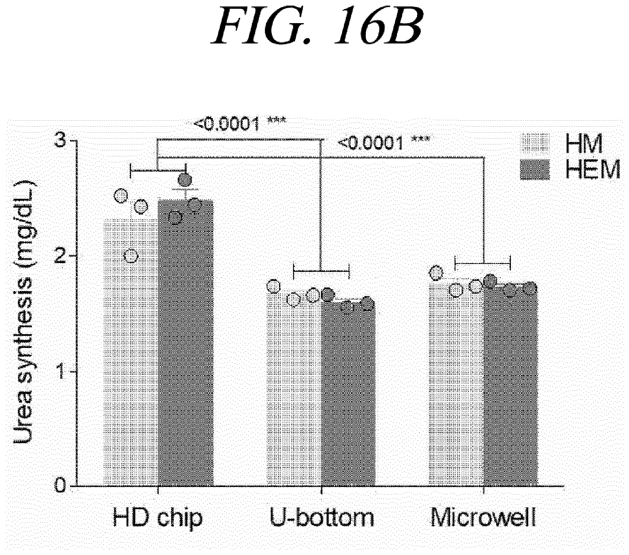

When the urea synthesis ability, an indicator of hepatic function, was compared between the groups, it can be seen from **FIG. 16B** that the urea synthesis ability of the liver organoid cultured in the HD chip was significantly increased compared to the liver organoids cultured in the systems of the control groups.

Therefore, it was confirmed that the HD chip can fabricate a liver organoid with improved functionality compared to the conventional organoid culture systems, the U-bottom well-plate and the microwell.

Test Example 6-5. Comparison of marker expression in long-term culture of human iPSC-derived liver organoid

Human iPSC-derived liver organoids were cultured for 10 days, 15 days and 30 days in respective culture systems and then, the marker expression was compared by immunostaining.

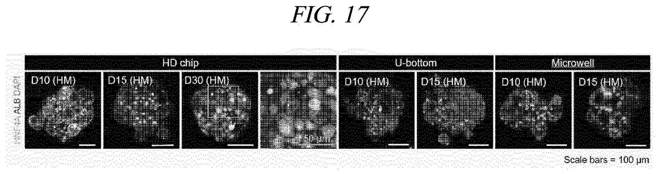

As a result, as shown in **FIG. 17****,** the shape of the liver organoid cultured in the HD chip was maintained uniformly for a long time compared to the control groups, the U-bottom plate and the microwell, and mature hepatic differentiation markers (intermediate and late markers), HNF4A and ALB, were expressed at higher levels. Also, it was confirmed that the liver organoids cultured in the U-bottom plate and the microwell for a long time of 30 days significantly decreased, whereas the liver organoid cultured in the HD chip formed a liver tissue-specific structure (high-level magnification images of the HD chip group) and was more mature.

### Test Example 7: Fabrication of high-throughput HD chip

### Test Example 7-1. Fabrication of high-throughput HD chip

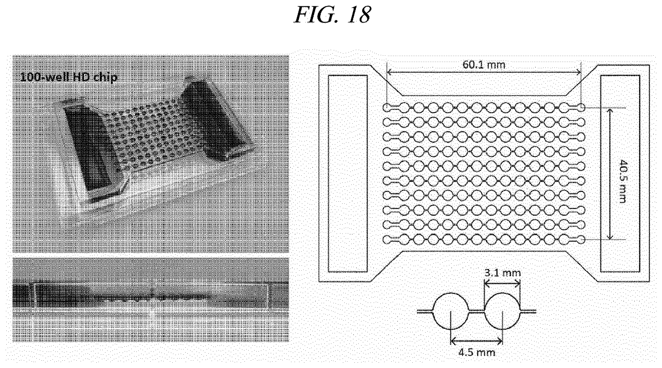

To improve the culture efficiency of a 25-well HD chip, a high-throughput HD chip having a 384-well plate size with an increased number of culture chambers like the existing 25-well HD chip was fabricated (**FIG. 18**).

The high-throughput HD chip can simultaneously perform hanging drop culture of spheroids/organoids in 100 wells. Also, like the existing 25-well HD chip, the high-throughput HD chip was designed to overlap each other in a vertical direction and to be put on a tray plate for culture.

A simulation was performed to check a fluid flow pattern in the high-throughput HD chip. Specifically, a time-dependent study was performed as a simulation under the condition of 10 rpm.

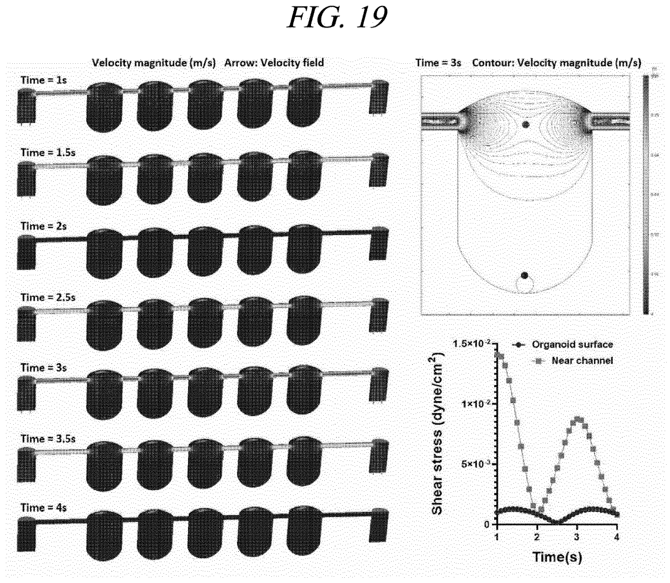

It can be seen that the fluidity of the fluid is relatively high in the vicinity of the channel, whereas the shear stress on the surface of the organoid is low (FIG. 19).

This shows that the HD chip is suitable for culturing a delicate organoid because the HD chip enables material exchange through a continuous flow for smooth culture medium circulation and is not directly damaged by overflow or flow of the organoid to other chambers.

A simulation was performed to compare the oxygen transmissibility from the U-bottom plate and the HD chip to the inside of the organoid. As a result of simulation in a steady state with the average flow, average values throughout culture were simulated.

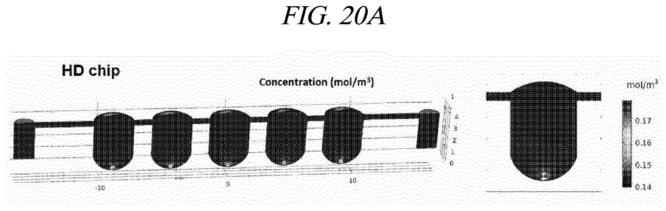

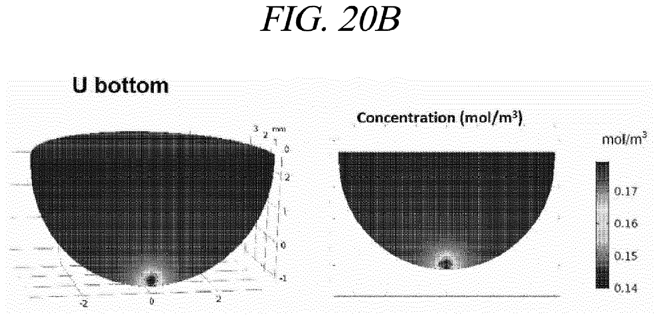

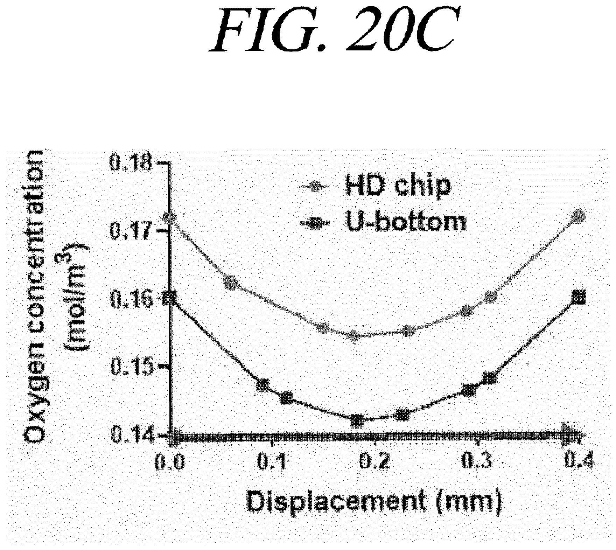

The oxygen concentration in the HD chip (**FIG. 20A**) and the oxygen concentration in the U-bottom plate well **(****FIG. 20B**) are shown in three-dimensional (left) and cross-sectional (right) images, and an oxygen concentration graph (**FIG. 20C**) corresponding to red arrows in the respective cross-sectional images was derived.

As a result of simulation, the oxygen concentration in the center of the organoid was higher when the organoid was cultured in the HD chip than when the organoid was cultured in the U-bottom plate. Therefore, it is predicted that the HD chip culture system will be more effective in culturing an organoid for which it is important to transfer a material to the center where cells are gathered at a high density.

### Test Example 7-2. Analysis of uniformity of human iPSC-derived liver organoid produced in high-throughput chip (100-well HD chip)

To check whether a uniform mass production of human iPSC-derived liver organoids is possible in a high-throughput 100-well HD chip, a total of 600,000 cells per chip were seeded by adjusting the number of cells to 6000 per well at a ratio of iPSC hepatic cells (H): vascular endothelial cells (E): mesenchymal stem cells (M) = 10: 7: 2. To check whether the additionally formed liver organoids have proliferative capacity, immunostaining for Ki67 was performed.

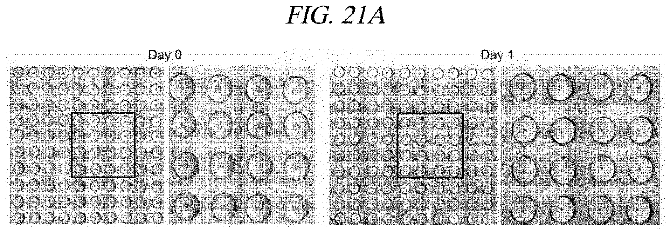

As a result, as shown in **FIG. 21A****,** when 600,000 cells were seeded into the HD chip, the cells were uniformly gathered and aggregated in culture medium droplets inside all the wells, and 100 organoids were all formed in a uniform size within 24 hours.

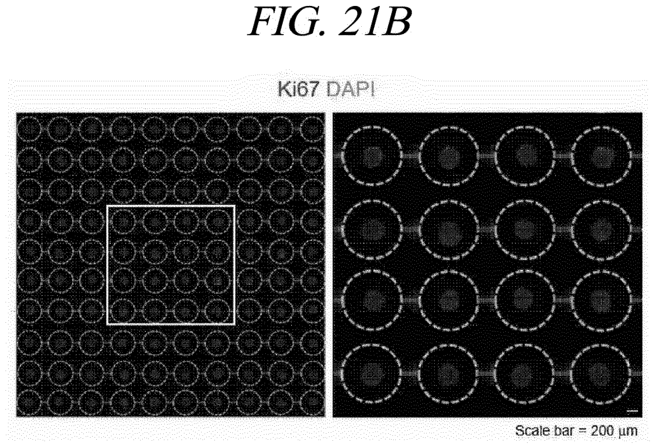

When a total of 100 organoids each formed per well were stained with Ki67, a cell proliferation marker, it was confirmed that active cell proliferation occurred in the organoids at a uniform level **(****FIG. 21B****).**

Therefore, it can be expected that the HD chip will be more suitable for simultaneous mass production of uniform liver organoids compared to the conventional organoid culture systems, the U-bottom well plate and the microwell.

Test Example 7-3. Comparison of uniformity of gene expression in human iPSC-derived liver organoid cultured in high-throughput HD chip

The uniformity of gene expression of a hepatocyte marker was compared between a liver organoid (HEM) cultured in the conventional U-bottom well plate and 100 human iPSC-derived liver organoids (HEM) fabricated in the high-throughput 100-well HD chip while simultaneously performing hanging drop culture of the 100 human iPSC-derived liver organoids (HEM).

As a result, as shown in **FIG**. **22**, when quantitative PCR analysis was performed on representative hepatic differentiation markers AFP, HNF4A and ALB, the gene expression was more significantly uniform with less deviation in the liver organoids fabricated by using the HD chip than in the liver organoid cultured in the conventional U-bottom well plate. Accordingly, it was verified that liver organoids with uniform expression of major differentiation markers can be produced through high-throughput hanging drop culture using the HD chip.

### Test Example 7-4. Mass production of human iPSC-derived normal and nonalcoholic steatohepatitis organoids and functional analysis

Human iPSC-derived liver organoids (HEMKS) were fabricated in the high-throughput 100-well HD chip. Then, a normal group was cultured in a normal culture medium for 7 days, whereas a nonalcoholic steatohepatitis (NASH) group was normally cultured for 5 days and then further cultured for 2 days in the culture medium mixed with 500 µM oleic acid, a free fatty acid, to induce steatohepatitis. After the organoids were cultured for a total of 7 days, the degree of fat accumulation was compared between the normal group and the steatohepatitis group, and CYP3A4 activity, one of the important indicators of hepatic function, was also compared. For more accurate steatohepatitis modeling and drug screening, liver organoids containing immune cells (Kupffer cells) and mesenchymal cells ((hepatic stellate cells) constituting a liver tissue microenvironment were fabricated. To this end, human iPSC-derived liver organoids were fabricated at a ratio of iPSC hepatic cells (H): vascular endothelial cells (E): mesenchymal stem cells (M): iPSC-derived Kupffer cells (K): iPSC-derived hepatic stellate cells (S) = 10:7:2:2:1 (HEMKS).

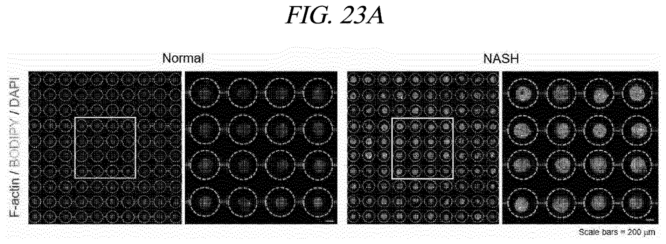

As a result, as shown in **FIG. 23A****,** it was confirmed that an actin filament (F-actin) was well distributed in the mass produced normal liver organoid and a BODIPY fluorescence signal labeling lipid was hardly observed. However, it was confirmed that the F-actin structure was abnormally observed from the NASH organoid and lipid accumulation occurred in the organoid.

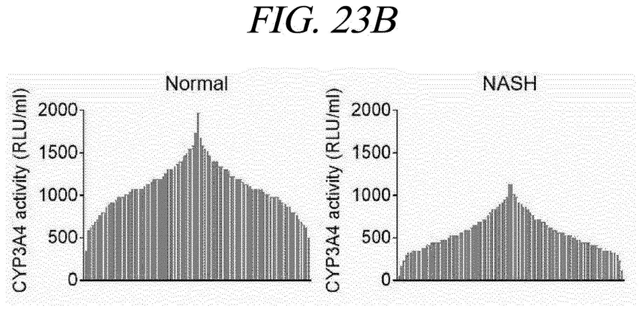

When CYP3A4 activity, an indicator of hepatic function, was compared, it was confirmed that the normal organoid group showed about 2 times higher activity than the NASH-induced organoid group, as shown in **FIG. 23B****,** which confirmed that the production of NASH organoids with impaired hepatic function caused by induction of steatohepatitis was successful.

Therefore, it was confirmed that the high-throughput HD chip can mass produce nonalcoholic steatohepatitis organoids exhibiting disease phenotypes as well as normal liver organoids.

### Test Example 7-5. Mass production of human iPSC-derived nonalcoholic steatohepatitis organoid and effective drug test

Human iPSC-derived liver organoids (HEMKS) were fabricated in the high-throughput 100-well HD chip. Then, a normal group was cultured in a normal culture medium for 7 days, whereas a nonalcoholic steatohepatitis (NASH) group was normally cultured for 5 days and then further cultured for 2 days in the culture medium mixed with 500 µM oleic acid, a free fatty acid, to induce steatohepatitis. Ezetimibe (Eze), an effective drug for treatment of fatty liver, is a drug candidate group that serves as a cholesterol absorption inhibitor and is conventionally used to treat high blood cholesterol and lipid abnormalities. After 5 days of normal culture, oleic acid (500 µM) and ezetimibe (50 µM) were mixed in the culture medium, followed by additional culture for 2 days to test the drug for steatohepatitis. After a total of 7 days of culture, the degree of fat accumulation was compared and the maker expression and gene expression were compared between groups by immunostaining and quantitative PCR analysis. For more accurate steatohepatitis modeling and drug screening, liver organoids containing immune cells (Kupffer cells) and mesenchymal cells (hepatic stellate cells) constituting a liver tissue microenvironment were fabricated. To this end, human iPSC-derived liver organoids were fabricated at a ratio of iPSC hepatic cells (H): vascular endothelial cells (E): mesenchymal stem cells (M): iPSC-derived Kupffer cells (K): iPSC-derived hepatic stellate cells (S) = 10:7:2:2:1 (HEMKS).

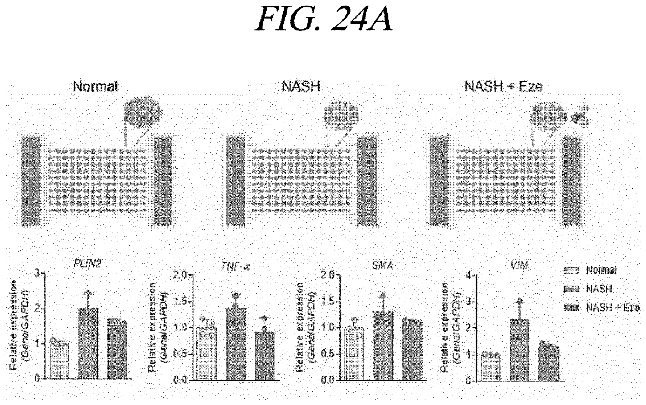

As a result, as shown in **FIG. 24A****,** when the gene expression in each of Normal, NASH and NASH + Eze organoid groups cultured in the HD chip was checked by quantitative PCR analysis, the expression levels of PLIN2, a fatty acid accumulation marker, TNF-α, an inflammation marker, and SMA and VIM, liver fibrosis markers, increased in the NASH group and decreased in the Eze-treated group.

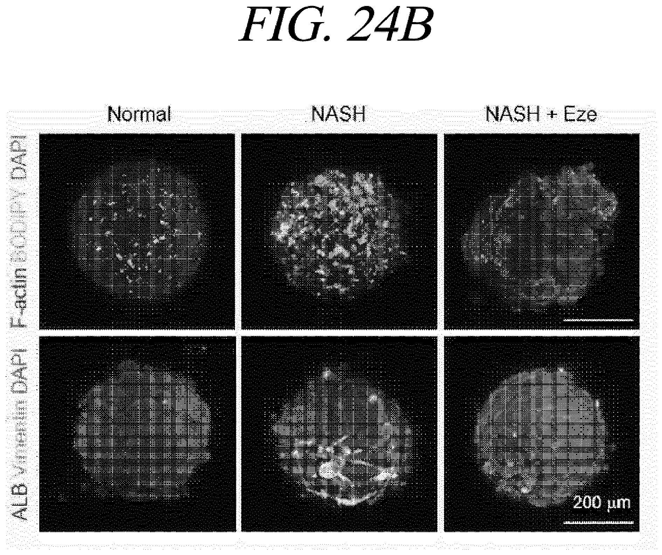

Also, as shown in **FIG. 24B****,** it was confirmed that F-actin, an actin filament marker, was well distributed in the normal organoid and a BODIPY fluorescence signal labeling lipid was hardly observed. Further, it was confirmed that the F-actin structure was observed from the NASH organoid and a large amount of lipid accumulation occurred in the organoid. It was confirmed that in the group treated with the effective drug (Eze), fatty acid accumulation was decreased, and, thus, functionality was recovered. The expression level of ALB, a hepatic differentiation marker, was decreased in the NASH organoid compared to the normal group, but recovered to a certain level in the Eze-treated group. Also, it was confirmed that the expression of vimentin, a liver fibrosis marker, was significantly increased in the NASH organoid group. Particularly, it was confirmed by immunostaining that hepatic stellate cells contained in the liver organoid were activated, and, thus, myofibroblasts that induce fibrosis were distributed in the organoid.

Therefore, it was confirmed that a human liver organoid fabricated based on the high-throughput HD chip can be used for nonalcoholic steatohepatitis modeling with a well-maintained disease phenotype and can also be used for drug efficacy screening.

Test Example 7-6. Mass production of human iPSC-derived nonalcoholic steatohepatitis organoid and ROS quantitative analysis

To check the accumulation of reactive oxygen species (ROS) caused by oxidative stress in Normal, NASH and NASH + Eze human iPSC-derived liver organoids (HEMKS) fabricated in the high-throughput 100-well HD chip by the same culture method as described above, ROS activity and quantitative analysis between the groups was performed by CM-H₂DCFDA staining, which is oxidative stress detection assay.

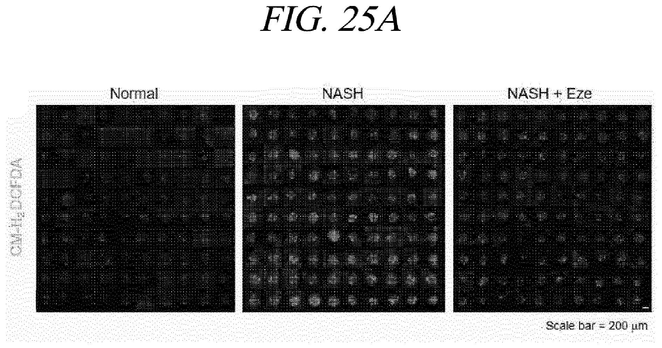

As a result, as shown in **FIG. 25A****,** oxidative stress caused by ROS was hardly observed in 100 mass produced normal organoid group, whereas ROS-related oxidative stress was significantly increased in the NASH organoid. Also, oxidative stress was decreased to a certain level or less in the NASH organoid group treated with the effective drug (Eze). This confirmed the possibility of highefficiency screening based on a HD chip liver organoid for evaluation of efficacy of the drug for steatohepatitis.

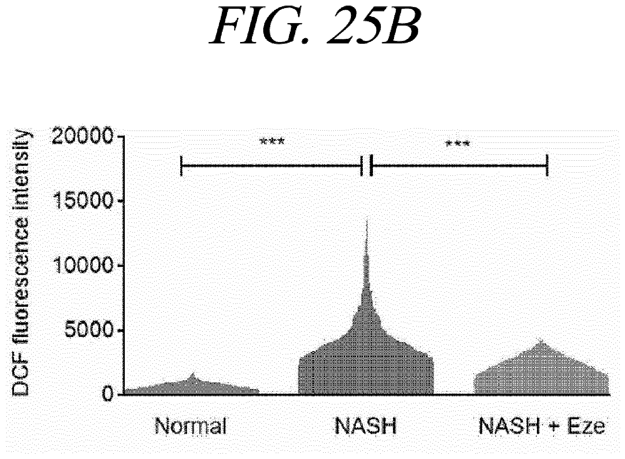

Also, as shown in **FIG. 25B****,** for quantitative evaluation of ROS oxidative stress in each organoid, the HD chip for culturing liver organoids was applied to a commercial plate reader, followed by on-chip analysis. The high-throughput HD chip was designed and fabricated with the same dimensions as a commercially available conventional 384-well plate. Thus, the high-throughput HD chip is compatible with conventional analysis devices such as a plate reader. As a result of quantitative analysis, the fluorescence intensity was significantly increased in the NASH organoid, and the fluorescence intensity was decreased in the Eze-treated organoid group, which confirmed that oxidative stress in the steatohepatitis organoid can be reduced by drug treatment.

Therefore, it was confirmed that the high-throughput HD chip fabricated with the same dimensions as the conventional 384-well plate can be used for large-scale drug screening and efficacy quantitative evaluation based on mass produced nonalcoholic steatohepatitis organoids.

### Test Example 8: Result of study on culture of human iPSC-derived pancreatic organoid

### Test Example 8-1. Scalable culture of human iPSC-derived pancreatic organoid (Comparison with conventional microwell and U-bottom well-plate methods)

Human iPSC-derived pancreatic organoids were cultured by using the fabricated HD chip. The U-bottom well-plate and the microwell which have been most widely used for production of 3D organoids were used as control groups.

For pancreatic organoids, iPSC-derived pancreatic progenitor cells (P), vascular endothelial cells (E), and mesenchymal stem cells (M) were mixed at a ratio of 10: 7: 2 and cells were seeded by adjusting the number of cells to 6000 per well. After the organoids were formed, additional differentiation was induced by the pancreatic organoids in a beta cell differentiation-inducing culture medium.

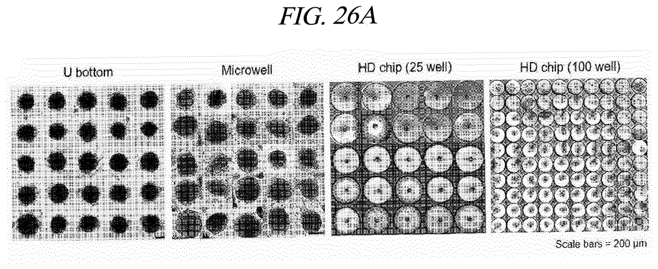

As a result, as shown in **FIG. 26A**, it was confirmed that the pancreatic organoids cultured in the HD chip were more uniformly formed into a regular shape than the control groups.

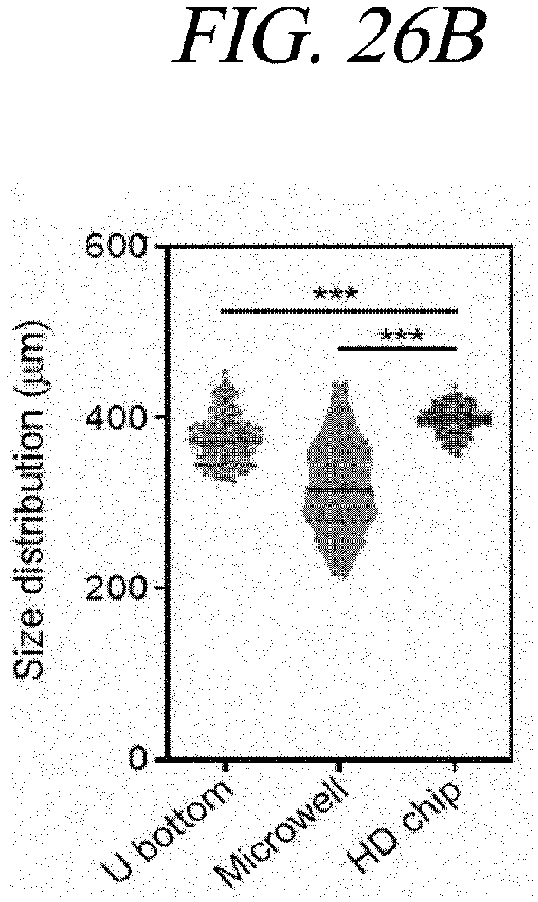

Further, as shown in **FIG. 26B**, when a size of the organoid formed in each culture environment was measured on day 2 of organoid formation, the pancreatic organoids fabricated in HD chip showed the smallest individual variation compared to the control groups. Accordingly, it can be seen that pancreatic organoids having a more uniform size can be fabricated through HD chip culture.

### Test Example 8-2. Comparison of marker expression and differentiation ability of human iPSC-derived pancreatic organoid

Human iPSC-derived pancreatic organoids (PEM) containing vascular endothelial cells were cultured for 5 days in respective culture systems and then, the expression of pancreatic differentiation markers and endothelial cell markers were compared between groups by immunostaining and gene expression analysis using qPCR.

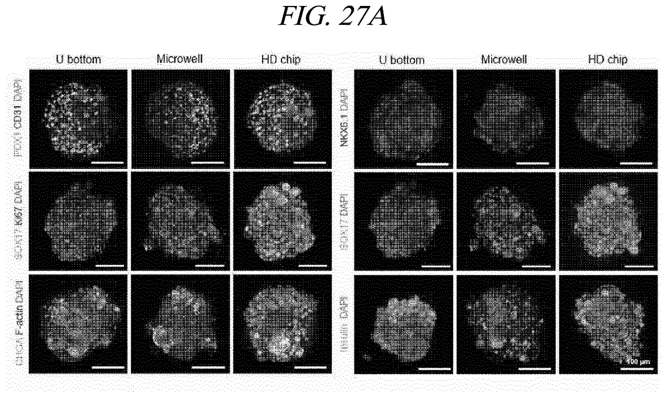

As a result, as shown in **FIG. 27A**, the expression levels of SOX17, an endoderm differentiation marker, PDX1 and NKX6.1, pancreatic progenitor cell markers, and CHGA and insulin, beta cell markers, were higher and the expression of CD31, a endothelial cell marker, was further improved in the pancreatic organoids cultured in the HD chip than in the control groups, the U-bottom plate and the microwell.

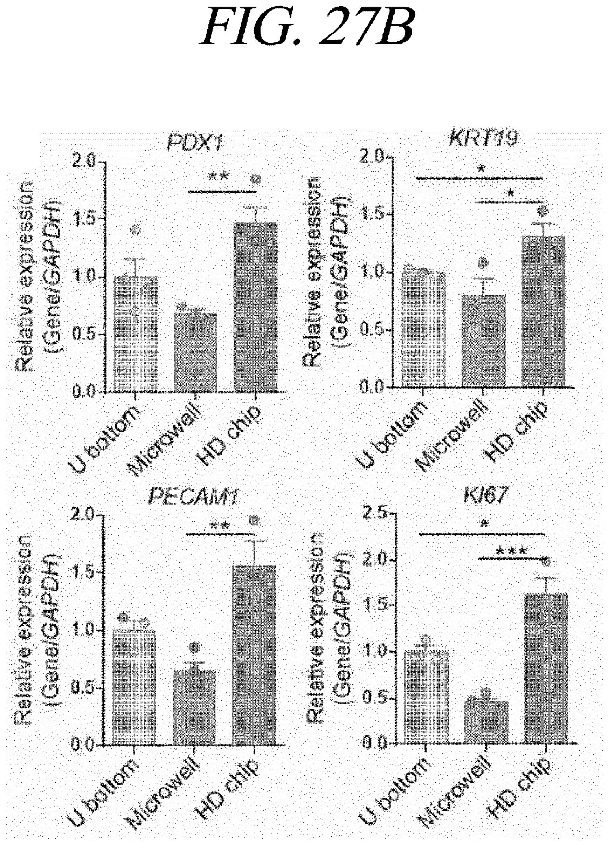

Also, as shown in **FIG. 27B**, when the gene expression level was compared between the control groups and the pancreatic organoids cultured in the HD chip, the expression of pancreatic differentiation markers (PDX1, KRT19), endothelial cell markers (PECAM1), and proliferation-related markers (KI67) tended to increase most in the HD chip group.

Accordingly, it was confirmed that the fabricated HD chip is a device capable of improving the uniformity of pancreatic organoids as well as the expression level of pancreas-specific differentiation markers compared to commercially available conventional platforms.

### Test Example 8-3. Comparison of insulin production of human iPSC-derived pancreatic organoid

Human iPSC-derived pancreatic organoids (PEM) were fabricated in a high-throughput 100-well HD chip and further cultured for 5 days in a beta cell differentiation-inducing culture medium, followed by immunostaining to confirm the uniformity of the organoids and the production of beta cell-specific insulin.

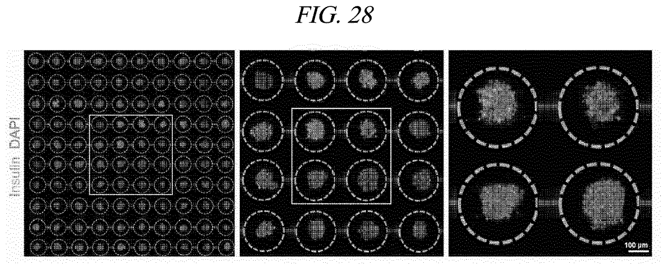

As a result, as shown in **FIG. 28**, human iPSC-derived pancreatic organoids, like liver organoids, could be mass produced uniformly in the 100-well HD chip, and 100 pancreatic organoids in which differentiation was induced in a beta cell culture medium produced insulin at a uniform and high level.

This shows that uniform human pancreatic organoids with the most important insulin production/secretion ability for treatment of diabetes can be produced through high-throughput HD chip culture.

Test Example 9: Fabrication of multi-organoid through high-throughput fusion by HD chip

### Test Example 9-1. Spheroid fusion through chip-to-chip transfer

The HD chip (25 well ver.) with an open top was designed to overlap each other in a vertical direction. Thus, it was confirmed that the HD chips can be orthogonally connected to each other.

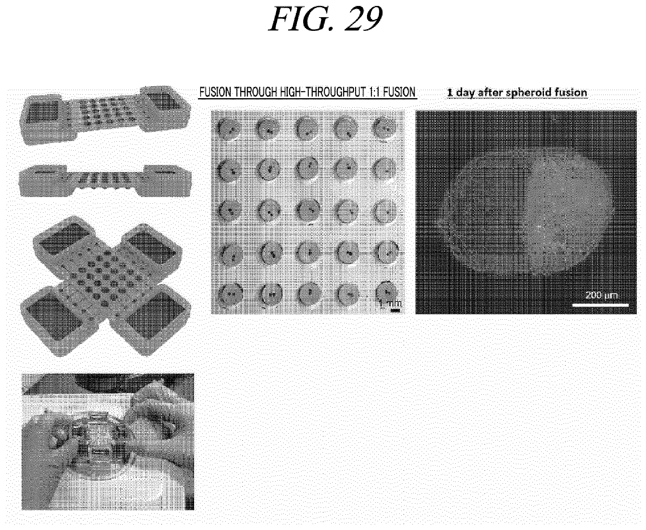

After different spheroids were cultured in respective HD chips, the two chips were connected one-to-one at once to induce spheroid transfer from one chip to the other **(****FIG. 29**; left).

Due to transfer from one HD chip to the other HD chip, two spheroids collected in one chip were located at the vertices of the concave parts of the culture medium droplets in wells, which confirmed that the spheroids can be fused efficiently **(****FIG. 29****;** right).

Recently, research to produce more advanced organoids through fusion of different organoids has been actively conducted. Thus, the HD chip can be used for this purpose and applied as a culture system for production of organoids with multiple tissue structures.

### Test Example 9-2. Comparison with U-bottom well-plate fusion method

After human iPSC-derived liver organoid (HEMKS) and pancreatic organoid (PEM) were cultured in respective HD chips, the two chips were connected one-to-one in a vertical direction to induce organoid transfer from one chip to the other. Then, a liver-pancreas fused organoid was fabricated through fusion of the organoids.

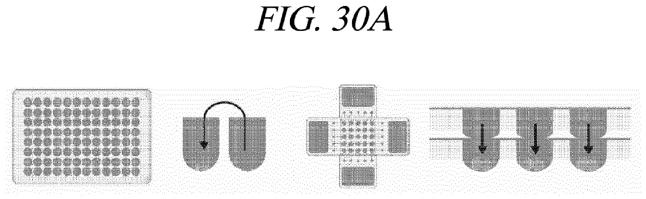

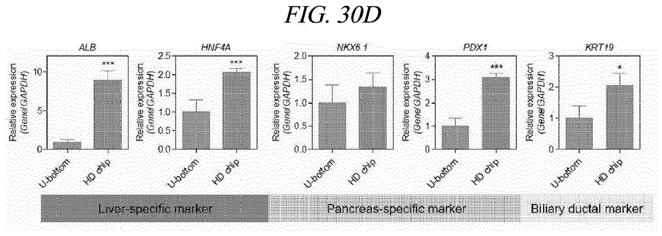

In the case of organoid fusion using the control group, the U-bottom plate, each cultured organoid needs to be transferred one by one at a ratio of 1:1 and then fused to each other **(****FIG. 30A****;** left). Meanwhile, in the case of organoid fusion using the HD chip, organoids in all wells can be transferred efficiently at once and then fused to each other **(****FIG. 30A****;** right).

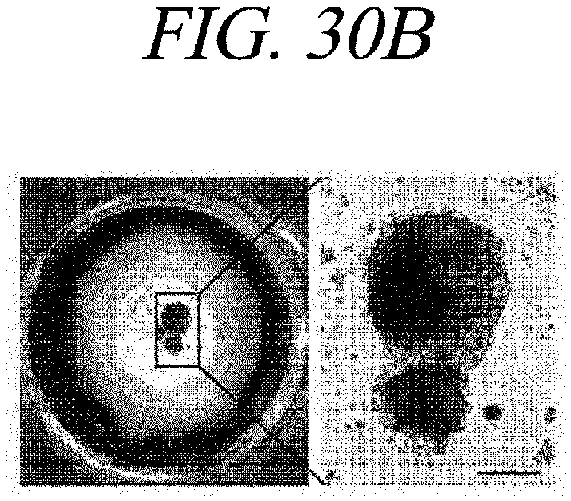

It was confirmed that the hepatic-pancreatic organoid cultured in the HD chip formed a fused organoid within 24 hours after transfer **(****FIG. 30B****).**

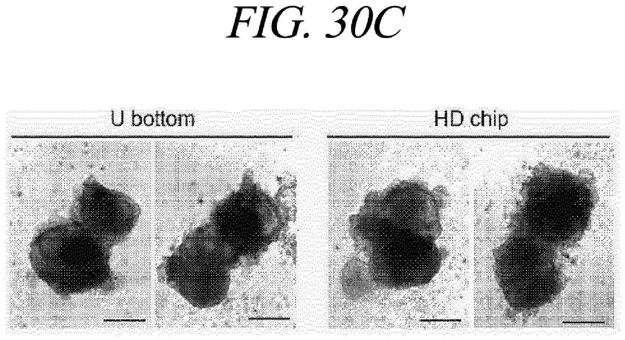

It was confirmed that hepatic-pancreatic organoids were formed in both the U-bottom plate, which is the control group, and the HD chip **(****FIG. 30C****).**

When the gene expression levels of hepatic and pancreatic differentiation markers were compared by qPCR analysis, the expression of hepatic differentiation markers (ALB, HNF4A), pancreatic differentiation markers (NKX6.1, PDX1) and a hepato-pancreatic bile duct marker (KRT19) was significantly increased in the HD chip group compared to the control group (U-bottom plate) (**FIG**. **30D**).

### Test Example 9-3. Confirmation of result of high-throughput HD chip-based spheroid fusion

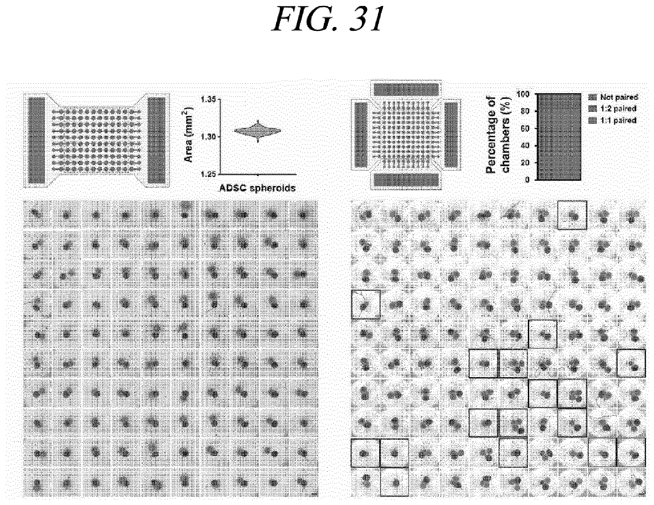

As a result of forming spheroids using ADSCs in the high-throughput HD chip, it was confirmed that 100 spheroids having a uniform size can be formed and cultured at once (**FIG. 31**; left).

As a result of overlapping two 100 well HD chips in the vertical direction to transfer spheroids to one side like the conventional 25 well HD chips, the spheroids were transferred with a success rate of 80% or more. Further improvement is expected if a material of the chips is changed later (**FIG. 31**; right).

### Test Example 9-4. High-throughput fusion of hepatic-pancreatic organoid based on HD chip

Human iPSC-derived liver organoids (HEMKS) were uniformly mass produced in the high-throughput 100-well-based HD chip, and human iPSC-derived pancreatic organoids (PEM) were uniformly mass produced in another HD chip. Then, chip-to-chip fusion was performed to overlap the two HD chips in the vertical direction and thus induce one-to-one fusion of the liver organoids and the pancreatic organoids.

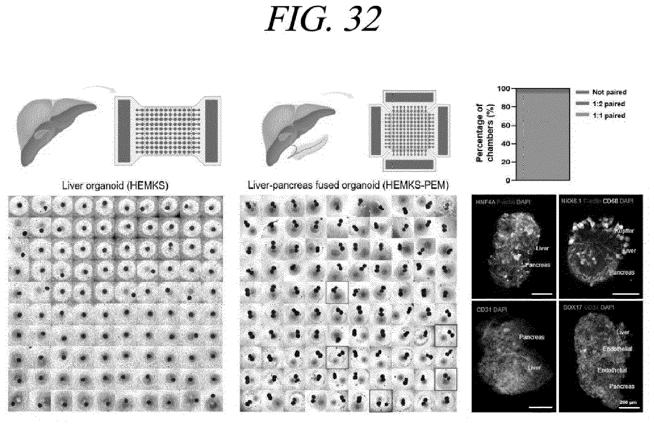

As shown in **FIG. 32****,** as a result of transferring the liver organoid toward the pancreatic organoid by overlapping two 100-well HD chips in the vertical direction, the liver organoids were transferred at a success rate of 95% or more. Therefore, it was confirmed that high-throughput mass production of liver-pancreatic multi-organoids through efficient organoid fusion was possible in addition to mass production of single organoids.

When immunostaining was performed to analyze each organ-specific differentiation marker of the formed liver-pancreatic fused multi-organoid, it was confirmed that HNF4A, a liver-specific differentiation marker, was expressed only in the liver organoid part, but not in the pancreatic organoid part. It was also confirmed that a Kupffer cell marker (CD68), a liver immune cell, was expressed only in the liver organoid, and NKX6.1, a pancreatic differentiation marker, was specifically expressed only in the pancreatic organoid part. Further, it was confirmed that SOX17, an endoderm marker expressed in both liver and pancreas, and CD31, a vascular marker, were well expressed in the multi-organoid fused in the HD chip.

### Test Example 10: Transplantation of liver organoid cultured in HD chip into mouse steatohepatitis model

To confirm the possibility of in vivo transplantation and steatohepatitis treatment effect of human iPSC-derived liver organoids (HEM-hepatocytes (H): vascular endothelial cells (E): mesenchymal stem cells (M)=10:7:2) mass produced in the HD chip, liver organoids were transplanted into a mouse NASH model in which steatohepatitis was induced with MCD (methionine-choline-deficient) feed. MCD diet deficient in methionine and choline is feed that induces steatohepatitis, oxidative stress, inflammation, and fibrosis and thus can induce lesions of nonalcoholic steatohepatitis (NASH). Therefore, the MCD diet is often used to induce a mouse steatohepatitis model.

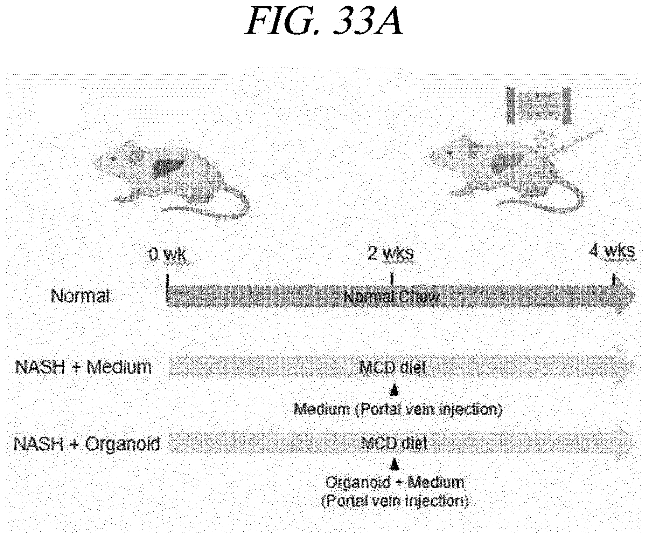

The normal group (Normal) was raised with normal chow for 4 weeks, and the steatohepatitis-induced group (NASH) was raised with the MCD feed for 4 weeks. After the first 2 weeks, only a liver organoid culture medium (100 µL) was injected through hepatic portal vein, and NASH was induced while feeding the MCD feed continuously for the remaining 2 weeks. The group (NASH+Organoid) transplanted with the liver organoids into the steatohepatitis-induced mice was raised with the MCD feed for 4 weeks. After the first 2 weeks, liver organoids produced in the HD chip were injected through hepatic portal vein (as being contained in 100 µl of the culture medium) and NASH was continuously induced while feeding the MCD feed for the remaining 2 weeks. A total of 400 liver organoids produced in 4 HD chips were collected in an insulin syringe and transplanted into each mouse through hepatic portal vein (**FIG. 33A**).

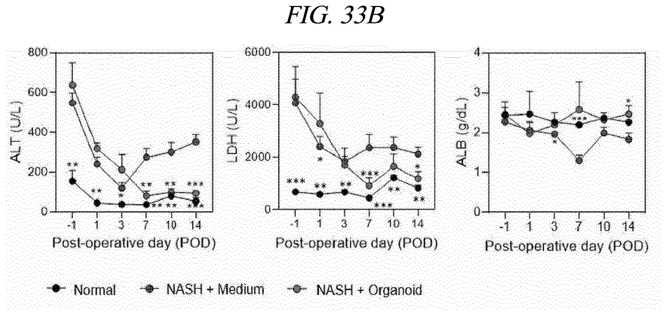

As shown in **FIG. 33B****,** as a result of blood analysis for each group after model induction, ALT and LDH levels, indicators of hepatotoxicity, measured on the day before organoid transplantation at week 2 of model induction (post-operative day (POD)-1) were significantly higher in the NASH-induced mouse group by the MCD diet than in the normal group. Up to day 3 of injection of the culture medium and the organoids through hepatic portal vein, the organoid culture medium was injected into the NASH group and elements contained in the culture medium were helpful in recovering the liver function. Thus, ALT and LDH levels the NASH group were gradually recovered right after injection as in the NASH+Organoid group transplanted with the liver organoids. However, from day 7 of injection, the medium culture was not much effective in recovering the liver function. Thus, in the NASH group injected only with the culture medium, the liver function was gradually decreased (increase in ALT and LDH levels), but in the NASH group transplanted with the liver organoids, ALT and LDH levels were decreased to nearly normal levels, which confirmed a remarkable improvement in liver function. From day 3 after culture medium injection, ALB level, an indicator of liver function, in the NASH group was also gradually decreased and thus was degraded in liver function, whereas ALB level in the NASH group transplanted with the liver organoids was recovered to a normal level.

**Therefore,** it was confirmed from these results that uniform liver organoids mass produced in the HD chip can reduce liver damage and contribute to restoration of liver function when applied to a nonalcoholic steatohepatitis mouse model. That is, it was confirmed that the high-throughput HD chip can be used as an efficient culture system for mass production of organoid-based cell therapeutic agents for treatment of liver damage.

Human iPSC-derived liver organoids (HEM) mass produced in the HD chip were transplanted into the mouse steatohepatitis (NASH) model through hepatic portal vein. After 2 weeks of transplantation, histological analysis and immunostaining were performed to check whether the organoids can induce steatohepatitis treatment effect.

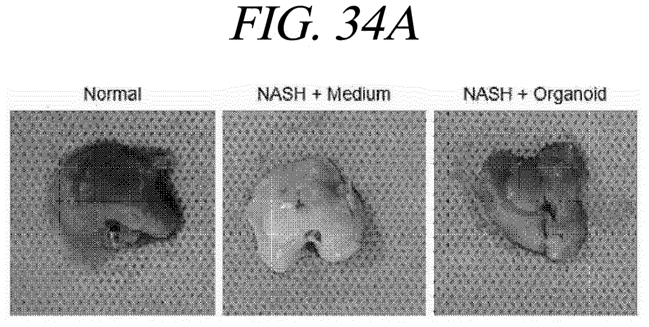

As a result, as shown in **FIG. 34A****,** the group injected with the culture medium at week 2 while steatohepatitis (NASH) was induced with the MCD feed for 4 weeks exhibited severe fatty liver lesions compared to the normal liver. However, in the group transplanted with the liver organoids produced in the HD chip, the recovery of some lesions was visually observed.

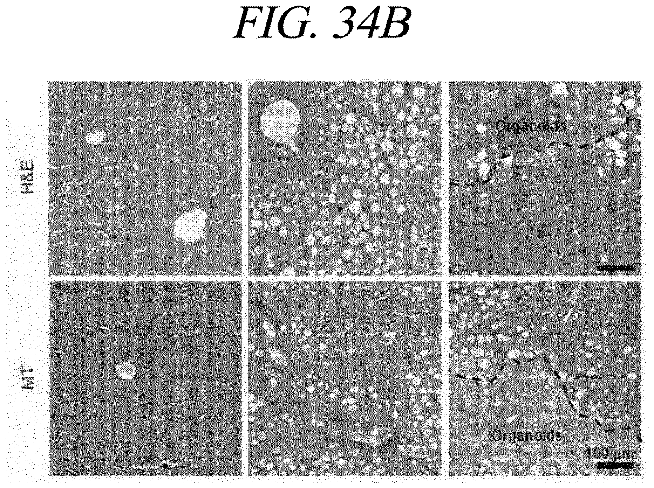

Also, as shown in **FIG. 34B****,** even when histological analysis was performed through H&E staining, it was confirmed that a greater amount of fat was accumulated in the NASH-induced model than in the normal liver. In the group injected with liver organoids, the transplanted liver organoids were engrafted in the area where fat was accumulated, and the fat accumulation was lessened and the damaged area was reduced. Even when staining for collagen accumulated due to fibrosis was performed through MT (Masson's Trichrome) staining, it was confirmed that fibrosis developed in some liver tissues in the NASH group injected only with the culture medium, whereas the collagen accumulation was lessened and the development of fibrosis was inhibited in the group transplanted with the liver organoids.

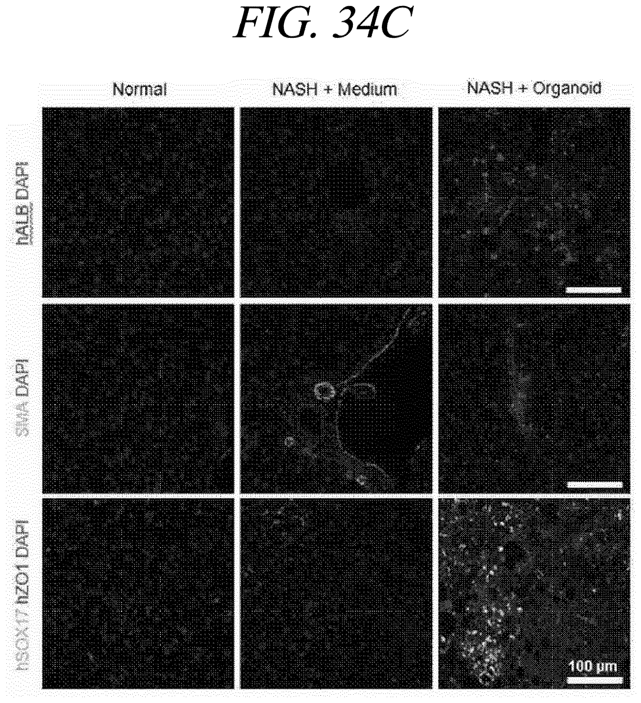

To check whether the transplanted liver organoids are well engrafted in fatty liver tissue and function well, immunostaining for hepatic differentiation markers (ALB, SOX17) and a tight junction marker (ZO1) was performed by using an antibody that reacts specifically only with human proteins, and it was confirmed that the human protein differentiation markers were expressed only in the mouse liver tissue transplanted with the human iPSC-derived organoids (FIG. 34C). Since the liver organoids were injected through hepatic portal vein in the present test, the transplanted organoids could be evenly distributed in the liver tissue. Therefore, the liver organoids are expected to be effective in recovering the liver function reduced by steatohepatitis. In the NASH model injected only with the culture medium, SMA-positive fibrosis was observed in the liver tissue, whereas in the NASH model treated with liver organoid transplantation, a fibrotic site where SMA was expressed was significantly reduced.

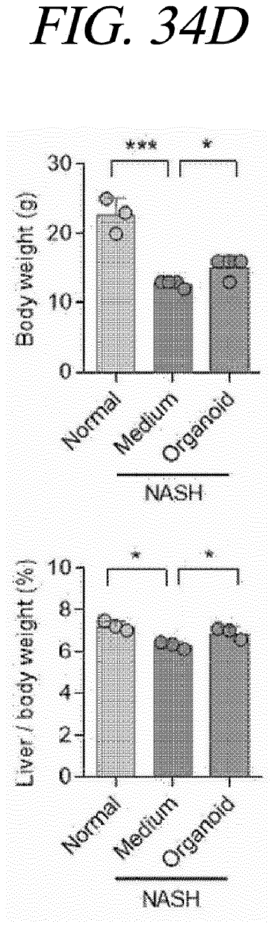

When the weights of mice in each group were measured 2 weeks after transplantation, the weight of the NASH mouse group injected only with the culture medium was significantly decreased, as previously known, whereas the weight of the NASH mouse group transplanted with the liver organoids was recovered to a certain level or more. recovered. Also, in the NASH group injected only with the culture medium, a liver:body weight ratio was measured lower than that of the normal group, whereas in the NASH group transplanted with the liver organoids, a liver:body weight ratio was recovered to a certain level **(****FIG. 34D****).** Therefore, it was confirmed that the transplanted liver organoids recovered the liver function inhibited by induction of NASH to a certain extent and reduced liver damage.

It was confirmed from these results that when uniform liver organoids mass produced in the HD chip can induce recovery of liver function and inhibit fibrosis when transplanted into a nonalcoholic steatohepatitis (NASH) mouse model. That is, the high-throughput HD chip can be used as an efficient culture system for mass production of organoid-based cell therapeutic agents for treatment of liver damage.

### Test Example 11: Additional application of HD chip

### Test Example 11-1. On-chip drug screening and fluorescence quantitative analysis using plate reader

Staining of live cells with calcein was performed for 10 minutes to label hADSC spheroids with fluorescence.

Calcein was injected at different concentrations into each line of a single chip, and, thus, hADSC spheroids cultured in the single HD chip were labelled to have different fluorescent intensities, followed by analysis by imaging the fluorescent intensities from the chip.

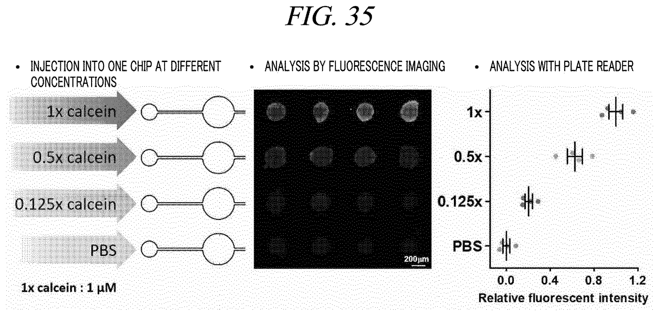

The HD chip (25 well ver.) fabricated in the present disclosure was designed with the same dimensions as a 384-well plate, it can be directly analyzed using a commercially available multi-use plate reader. That is, spheroids or organoids can be cultured in the chip and stained with fluorescence, immediately followed by measurement and quantification of fluorescent intensities with the plate reader (**FIG. 35**).

Actually, it can be seen that there is a high correlation between the result of fluorescence imaging and the result of measuring fluorescent intensities with a plate reader.

### Test Example 11-2. Confirmation of possibility to fabricate device by 3D printing

Although the above-described device of the present disclosure is fabricated with a PDMS polymer, there is a limitation in terms of mass production. Therefore, when a 3D printing process was applied to overcome the limitation, the possibility to fabricate the device of the present disclosure into a design to be actually commercialized by 3D printing was checked.

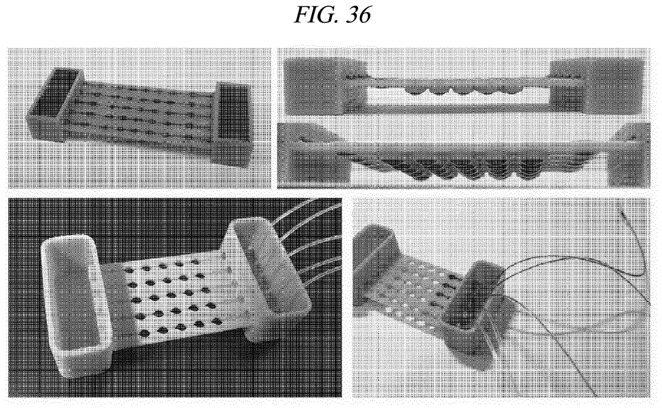

As a result, as shown in **FIG. 36****,** it was confirmed that the device of the present disclosure can be easily fabricated by 3D printing, and 3D printing facilitates a change in design. Also, 3D printing enables fabrication of the device of the present disclosure with plastic-based materials which are easy to standardize and mass produce.

Also, it is possible to culture organoids in a hanging drop culture device while reducing an individual variation through continuous mixing of a culture medium on a rocker without an external connection device during culture. Alternatively, a tube may be further connected and a syringe pump may be used if necessary depending on the purpose of a test or the type of analysis.

### Test Example 11-3. Utilization of accessory applicable to device of the present disclosure

An accessory capable of increasing the applicability of the device of the present disclosure was fabricated and the applicability was checked.

Specifically, besides a device culture method of the present disclosure for inducing gelation by simply adding a gel solution to a culture medium (**FIG. 6**), a ring-shaped accessory may be used to form the gel solution into a specific shape by using the viscosity and surface tension of the gel solution even before the gel solution touches the culture medium. Hydrogels with various properties can be used, and various types of gels used for spheroid/organoid culture can be applied (e.g., low-viscosity gels, gels that should not be in contact with culture medium before solidification).

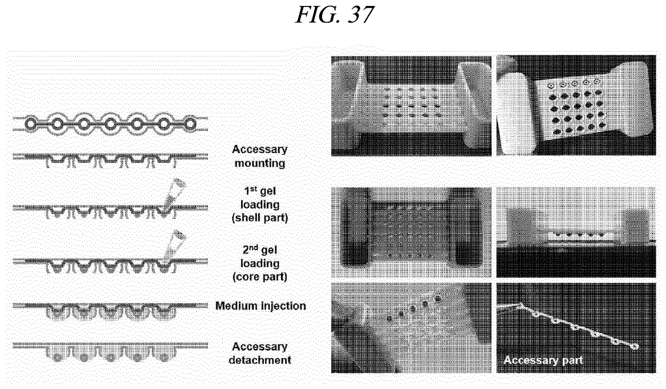

The accessory can be designed to fit the shape of the chip body, attached and detached. When the spheroids/organoids are well formed after time passes, the accessory can be removed, followed by continuous culture (**FIG. 37**).

To separately form a central part from an outer layer part during fabrication of a multilayer 3D spheroid by a conventional method, an additional microfluidic device is required, and in most cases, a multilayer spheroid having a small size is fabricated.

This conventional method shows a relatively high loss rate and is not suitable for culturing cell aggregates, such as large-sized spheroids or organoids.

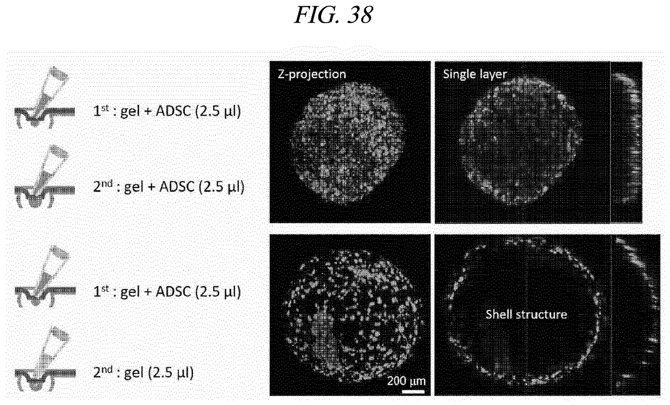

It was confirmed that the HD chip developed in the present disclosure can be further equipped with an accessory to separately inject a central part and an outer layer part, and, thus, a multilayer spheroid in the form of a capsule can be formed very easily and efficiently (**FIG. 38**).

The present disclosure has been described with reference to the preferred embodiments thereof. It can be understood by a person with ordinary skill in the art that the present disclosure can be implemented as being modified and changed departing from the spirit and the scope of the present disclosure. Accordingly, the above-described embodiments should be considered in descriptive sense only and not for purposes of limitation. Also, the technical scope of the present disclosure is defined not by the detailed description of the invention but by the appended claims, and all differences within the scope will be construed as being comprised in the present disclosure.

## Claims

1. A cell aggregate culture device, comprising:
a culture chamber including one or more wells;
one or more reservoirs storing a culture medium; and
a microchannel connecting the culture chamber and the reservoir.

2. The cell aggregate culture device of Claim 1,
wherein the culture chamber further includes a microchannel connecting a plurality of wells.

3. The cell aggregate culture device of Claim 1,
wherein a diameter of the well is 1.5 mm to 4 mm, and
a distance between the wells connected through the microchannel is 1.5 mm to 5 mm.

4. The cell aggregate culture device of Claim 1,
wherein the reservoir is located at both ends of the device.

5. The cell aggregate culture device of Claim 1,
wherein the cell aggregate is a spheroid or organoid derived from one of mesenchymal stem cells, neural stem cells, vascular endothelial cells, induced pluripotent stem cells, embryonic stem cells, tissue stem cells, fetal stem cells, cancer stem cells and cardiac cells.

6. The cell aggregate culture device of Claim 1,
wherein the cell aggregate is derived from one selected from the group consisting of brain, optic cup, kidney, liver, pancreas, neural tube, stomach, large intestine, prostate, breast, heart, salivary gland, endometrium, mammary gland, thyroid, tongue, small intestine, esophagus, spinal cord, skin, bile duct, lung, blood vessel, muscle, adrenal cortex and thyroid organoids.

7. A cell aggregate culture system, comprising:
the device of any one of Claim 1 to Claim 6;
a rocker; and
a culture medium shared through a microchannel.

8. The cell aggregate culture system of Claim 7,
wherein the rocker may cause the device to make a swing motion.

9. A method of culturing a cell aggregate by using the culture system of Claim 7.
